# EUROPEAN PATENT SPECIFICATION

(11) **EP 3 699 593 B1**
(45) Date of publication and mention of the grant of the patent: **18.06.2025**
(21) Application number: 20156118.0
(22) Date of filing: 07.02.2020
(51) Int. Cl.: G01N 33/543, G01N 33/58, G01N 33/68

(54) **METHOD FOR ACQUIRING INFORMATION ON ANALYTE**
VERFAHREN ZUR ERFASSUNG VON INFORMATIONEN AUF EINEM ANALYTEN
PROCÉDÉ D'ACQUISITION D'INFORMATIONS SUR UN ANALYTE

(30) Priority: 20.02.2019 JP 2019028495
(43) Date of publication of application: 26.08.2020
(73) Proprietor: SYSMEX CORPORATION, Kobe-shi Hyogo 651-0073 (JP)
(72) Inventor: Yamashita, Kazuto, Hyogo, 651-0073 (JP); Ganguly, Akshay, Hyogo, 651-0073 (JP); Saiki, Naoya, Hyogo, 651-0073 (JP); Iwanaga, Shigeki, Hyogo, 651-0073 (JP)
(74) Representative: Hoffmann Eitle

(56) References cited:
- EP-A1- 3 415 912
- WO-A2-2007/064972
- US-A1- 2010 261 292
- SOYEONG JU ET AL: "One-Shot Dual-Code Immunotargeting for Ultra-Sensitive Tumor Necrosis Factor-[alpha] Nanosensors by 3D Enhanced Dark-Field Super-Resolution Microscopy", ANALYTICAL CHEMISTRY, vol. 90, no. 8, 22 March 2018 (2018-03-22), US, pages 5100 - 5107, XP055698187, ISSN: 0003-2700, DOI: 10.1021/acs.analchem.7b05107
- ZHANG W I ET AL: "Super-Resolution Microscopy of Cerebrospinal Fluid Biomarkers as a Tool for Alzheimer's Disease Diagnostics", JOURNAL OF ALZHEIMER'S DISEASE, IOS PRESS, NL, vol. 46, no. 4, 26 June 2015 (2015-06-26), pages 1007 - 1020, XP009508121, ISSN: 1387-2877, DOI: 10.3233/JAD-150064

## Description

### TECHNICAL FIELD

The present invention is defined in the appended claims and relates to: a method for acquiring information on a polypeptide that is an analyte; and a method for capturing a polypeptide that is an analyte.

### BACKGROUND

In the pathological diagnosis and the decision of the strategy for a treatment, it is useful to acquire information on the quantity or structure of an analyte in a clinical specimen collected from a subject. For example, in Alzheimer's disease, the quantity and structure of an analyte, e.g., amyloid β (Aβ), vary with the evolution of the disease. Therefore, the clinical condition can be understood accurately by acquiring information on the quantity and structure of the analyte. Examples of a disease associated with the denaturation of a protein include, in addition to Alzheimer's disease, Huntington's disease, Parkinson's disease, prion disease and amyotrophic lateral sclerosis (ALS).

As one example of a method for acquiring information on the quantity or structure of an analyte in a clinical specimen, it is disclosed that Aβ can be detected by immobilizing Aβ contained in a cerebrospinal fluid (CSF) onto a cover glass, then immunostaining the Aβ by indirect immunofluorescence, and then imaging the Aβ with a super-resolving microscope in Non Patent Document 1. In an acquired image, Aβ is detected as a spot having a size and strength corresponding to the degree of aggregation.

### PRIOR ART

### NON PATENT DOCUMENT

Non Patent Document 1: Zhang W.I. et. al., Super-Resolution Microscopy of Cerebrospinal Fluid Biomarkers as a Tool for Alzheimer's Disease Diagnostics, J Alzheimers Dis, 2015, vol.46, p.1007-1020

### SUMMARY OF THE INVENTION

### PROBLEM TO BE SOLVED

In the method disclosed in Zhang W.I. et. al., Super-Resolution Microscopy of Cerebrospinal Fluid Biomarkers as a Tool for Alzheimer's Disease Diagnostics, J Alzheimers Dis, 2015, vol.46, p.1007-1020, a CSF is dropped onto a cover glass, and therefore a contaminant in the CSF, as well as Aβ, is also immobilized on the cover glass. Therefore, a primary antibody and a fluorescently labeled secondary antibody which are used in the immunostaining is sometimes bonded to the contaminant immobilized on the cover glass in a non-specific manner. Or the fluorescently labeled secondary antibody is sometimes bonded to the cover glass in a non-specific manner. When the contaminant and the cover glass, as well as Aβ, are also labeled with a labeling antibody, information on the quantity or structure of Aβ that is an analyte cannot be acquired with high accuracy.

In a clinical specimen, e.g., a CSF, collected from a subject, an analyte is not necessarily to occur in an enough quantity for detection. Therefore, for the measurement of an analyte in a clinical specimen, it is demanded to improve detection sensitivity. Actually, the present inventors have found that, even in a measurement system which can detect a synthetic polypeptide that is an analyte satisfactorily, when an analyte contained in a clinical specimen is measured, there is still a room for improvement with respect to detection sensitivity.

### MEASURE TO SOLVE THE PROBLEM

The present inventors have found that a polypeptide, which is an analyte, in a clinical specimen can be detected with higher sensitivity by using a capture substance capable of binding to a C-terminal region of the polypeptide in an immune complex transfer (ICT) method. This finding leads to the accomplishment of the present invention.

The present invention provides a method for acquiring information on an analyte, comprising the steps of: bringing a polypeptide that is the analyte into contact with a capture substance that binds to the polypeptide to form a complex comprising the polypeptide and the capture substance; bonding the capture substance in the complex to a first solid phase to immobilize the complex onto the first solid phase; collecting the first solid phase having the complex immobilized thereon; releasing the complex from the collected first solid phase, and then bonding the capture substance in the released complex to a second solid phase to immobilize the complex onto the second solid phase; and acquiring information on the polypeptide from the complex immobilized on the second solid phase, wherein a capture substance that binds to at least one of the first solid phase and the second solid phase binds to a C-terminal region of the polypeptide as further defined in the appended claims.

The present invention also provides a method for capturing an analyte, comprising the steps of: bringing a polypeptide that is the analyte into contact with a capture substance that binds to the polypeptide to form a complex comprising the polypeptide and the capture substance; bonding the capture substance in the complex to a first solid phase to immobilize the complex onto the first solid phase; collecting the first solid phase having the complex immobilized thereon; and releasing the complex from the collected first solid phase, and then bonding the capture substance in the released complex to a second solid phase to immobilize the complex onto the second solid phase, wherein a capture substance that binds to at least one of the first solid phase and the second solid phase binds to a C-terminal region of the polypeptide as further defined in the appended claims.

The present invention also provides a method for acquiring information on an analyte, comprising the step of acquiring information of a polypeptide that is the analyte from a complex comprising the polypeptide and a capture substance that binds to the polypeptide. In the method, the complex is produced by: bringing the polypeptide into contact with the capture substance to form a complex comprising the polypeptide and the capture substance; bonding the capture substance in the complex to a first solid phase to immobilize the complex onto the first solid phase; collecting the first solid phase having the complex immobilized thereon; and releasing the complex from the collected first solid phase, and then bonding the capture substance in the released complex to a second solid phase. In the method, a capture substance that binds to at least one of the first solid phase and the second solid phase binds to a C-terminal region of the polypeptide as further defined in the appended claims.

### EFFECT OF INVENTION

According to the present invention as defined in the appended claims, it is possible to acquire information on the quantity or the structure of a polypeptide that is an analyte with high accuracy.

### BRIEF DESCRIPTION OF THE DRAWINGS

Fig. 1 is a flow chart showing the processing procedure in the method for acquiring information on an analyte according to the present embodiment.
Fig. 2 is a schematic diagram showing the step of forming a complex and the step of immobilizing the complex onto a first solid phase.
Fig. 3 is a schematic diagram showing the step of collecting a complex and the step of releasing the complex.
Fig. 4A is a schematic diagram showing the step of immobilizing a complex onto a second solid phase.
Fig. 4B is a schematic diagram showing the step of immobilizing a complex onto a second solid phase.
Fig. 5 is a schematic diagram showing the step of forming a complex and the step of immobilizing the complex onto a first solid phase.
Fig. 6A is a schematic diagram showing the step of immobilizing a complex onto a second solid phase.
Fig. 6B is a schematic diagram showing the step of immobilizing a complex onto a second solid phase.
Fig. 7 is a schematic diagram showing the step of forming a complex and the step of immobilizing the complex onto a first solid phase.
Fig. 8 is a schematic diagram showing the step of collecting a complex and the step of releasing the complex.
Fig. 9A is a schematic diagram showing the step of immobilizing a complex onto a second solid phase.
Fig. 9B is a schematic diagram showing the step of immobilizing a complex onto a second solid phase.
Fig. 10 is a schematic diagram showing the distribution of a fluorescent dye in a light-emitting state in a complex immobilized on a second solid phase.
Fig. 11 is a flow chart showing the step of acquiring information.
Fig. 12 is a diagram showing the procedure for acquiring a super-resolved image and classifying bright points into groups in the information acquisition step.
Fig. 13 is a diagram showing an example of a screen displayed on a display unit of a detection device in the information acquisition step.
Fig. 14 is a diagram showing the configuration of a detection device for carrying out the information acquisition step automatically.
Fig. 15 is a fluorescent image of Aβ in a cerebrospinal fluid which is acquired in Example 1.
Fig. 16 is a fluorescent image of Aβ in a cerebrospinal fluid which is acquired in Example 2.
Fig. 17A is a super-resolved image of Aβ in a cerebrospinal fluid which is acquired in Example 2.
Fig. 17B is a super-resolved image of Aβ in a cerebrospinal fluid which is acquired in Example 2.
Fig. 18 is a graph showing the results of the measurement of Aβ in a cerebrospinal fluid in Example 3.
Fig. 19 is a graph showing the results of the measurement of a synthetic Aβ peptide in Example 3.

### DETAILED DESCRIPTION OF THE PREFERRED EMBODIMENTS

### (Complex formation step)

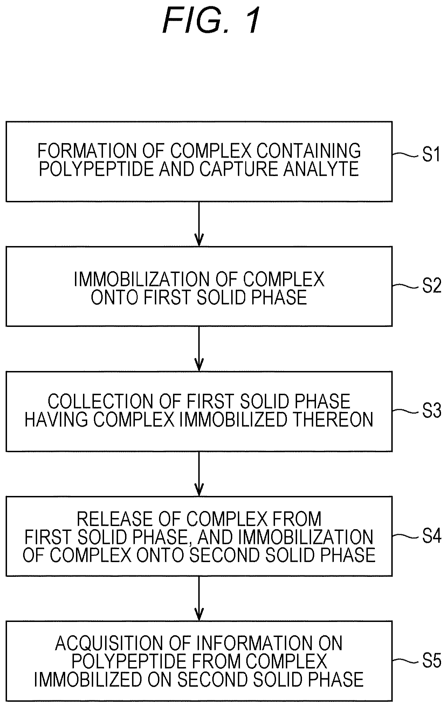

Referring to Fig. 1, the processing procedure in the method for acquiring information on an analyte (also simply referred to "method", hereinafter) according to the present embodiment is described. As shown in step S1, in the method according to the present embodiment, a polypeptide that is the analyte is brought into contact with a capture substance capable of binding to the polypeptide to form a complex containing the polypeptide and the capture substance.

In the method according to the present embodiment, the analyte is a polypeptide. The term "polypeptide" as used herein also includes a protein within the scope thereof. The polypeptide may be an artificially synthesized polypeptide or an organism-derived polypeptide that is contained in a biological sample or the like. A preferred example of the analyte is an organism-derived polypeptide. Examples of the biological sample include a clinical specimen collected from a living organism and cultured cells. Examples of the clinical specimen include: a body fluid such as a cerebrospinal fluid, blood (whole blood, plasma, serum), a tissue fluid and urine; and a tissue specimen such as a brain tissue.

Because the formation of the complex is generally carried out in a solution, it is preferred that the sample containing the polypeptide has a liquid form. The liquid sample is not limited to a solution, and may be a liquid suspension or a sol. In the case where a solid sample such as a tissue specimen is used, it is preferred to carry out a treatment for making the sample into a liquid form prior to the method according to the present embodiment. The type of the treatment can be selected appropriately from known methods depending on the type of the sample to be used. For example, in the case where the sample is a solid tissue, the solid tissue is homogenized in a solution containing a surfactant, then solid components including debris are separated from the resultant solution by centrifugation to obtain a supernatant containing a polypeptide.

The type of the polypeptide is not particularly limited, and the type of the polypeptide may be selected arbitrarily from polypeptides that cause diseases and disorders. Examples of the polypeptide of this type include Aβ, tau protein, huntingtin, prion and α-synuclein. Among these polypeptides, Aβ is particularly preferred. Aβ is a polypeptide normally composed of 39 to 43 amino acid residues. The term "amyloid β" or "Aβ" as used herein includes an Aβ polypeptide having any length, unless otherwise stated.

In the present embodiment, the polypeptide may have a form of a multimer. A multimer is also called as a "polymer", and a multimer is formed by the physical or chemical polymerization or aggregation of a plurality of monomeric polypeptides. The multimer is only needed to contain a plurality of monomeric polypeptides, and the multimer may also contain another molecule. In the multimer, it is not needed for the monomeric polypeptides to be bonded strongly to each other via a covalent bond. An aggregate that is a cluster of a plurality of monomeric polypeptides bonded to each other through a more moderate bond is also included within the scope of the multimer. For example, Aβ has a property of being aggregated to form an insoluble amyloid fibril. Examples of the multimer of the polypeptide include an Aβ oligomer formed by the polymerization of Aβ monomers and a tau oligomer formed by the polymerization of tau proteins.

The term "capture substance" as used herein refers to a substance which can bind to a polypeptide that is the analyte specifically and can be immobilized on a solid phase to capture the polypeptide onto the solid phase. Examples of the capture substance include an antibody and an aptamer. Hereinafter, an antibody that can be used as the capture substance is also referred to as a "capture antibody". The term "antibody" as used herein includes, within the scope thereof, an antigen-binding antibody fragment such as Fab, F(ab')₂ and Fab' and derivatives thereof. The capture antibody may be any one of a monoclonal antibody and polyclonal antibody, and the capture antibody is preferably a monoclonal antibody.

The term "solid phase" as used herein refers to an insoluble support for immobilizing the capture substance thereon. The wording "a capture substance is immobilized onto a solid phase" means that the capture substance and the solid phase are bonded to each other directly or indirectly so that the capture substance can be captured on the solid phase. When the capture substance that is bonded specifically to the polypeptide is immobilized onto the solid phase, the polypeptide is captured on the solid phase through the capture substance.

The method according to the present embodiment relies on the ICT method. As mentioned below, in the method, a complex of the capture substance and the polypeptide is transferred from a first solid phase to a second solid phase. According to this transfer of the complex, the influence by contaminants or the like is reduced, and therefore the polypeptide can be measured with higher sensitivity. A single type of the capture substance may be used, or two types of the capture substances may be used. In an exemplary embodiment in which a single type of the capture substance is used, the capture substance is one capable of binding to both of the first solid phase and the second solid phase. In an embodiment as defined in the appended claims in which two types of the capture substances are used, the capture substances are a combination of a capture substance capable of binding to the first solid phase (also referred to as a "first capture substance", hereinafter) and a capture substance capable of binding to the second solid phase (also referred to as a "second capture substance", hereinafter. Alternatively, it disclosed to use a combination of the first capture substance or the second capture substance and a capture substance capable of binding to both of the first solid phase and the second solid phase.

In the present embodiment as defined in the appended claims, a capture substance capable of binding to at least one of the first solid phase and the second solid phase can bind to a C-terminal region of the polypeptide. Preferably, the capture substance capable of binding to the first solid phase can bind to a C-terminal region of the polypeptide. The C-terminal region of a polypeptide does not have a critical boundary, and may be, for example, a region lying between an amino acid residue located at a position in the vicinity of the center and an amino acid residue located at the C-terminal in the amino acid sequence for the monomeric polypeptide. When the full length of the monomeric polypeptide is 2n residues or 2n+1 residue (wherein n represents an integer of 1 or more), the amino acid residue located in the vicinity of the center may be an amino acid residue at position-(n+1). For example, when the polypeptide is composed of 50 amino acid residue, the C-terminal region may be a region lying between position-26 and an amino acid residue located at position-50. Alternatively, when a specific C-terminal region of the polypeptide is defined by the structure, physical property, function or the like thereof in the art, it is possible to follow the definition. For example, in Aβ₁₋₄₀ and Aβ₁₋₄₂, it is known that a region lying between an amino acid residue located the N-terminal and an amino acid residue located at position-28 is hydrophilic and a region lying between an amino acid residue located at position-29 and an amino acid residue located at the C-terminal is hydrophobic. When the polypeptide is Aβ₁₋₄₀ or Aβ₁₋₄₂, it is possible to define a region lying between an amino acid residue located at position-29 and an amino acid residue located at the C-terminal as the C-terminal region.

In the present embodiment as defined in the appended claims, the capture substance capable of binding to the C-terminal region of the polypeptide may recognize an epitope present in the C-terminal region of the polypeptide or the three-dimensional structure of the C-terminal region of the polypeptide to bind specifically to the polypeptide. Hereinafter, the epitope or the three-dimensional structure recognized by the capture substance is also referred to as a "recognition site of the capture substance". For example, in the case where the polypeptide is Aβ (particularly Aβ₁₋₄₂), an antibody capable of recognizing an epitope present in a region lying between an amino acid residue located at position-22, preferably position-29, more preferably position-33, in Aβ and an amino acid residue located at the C-terminal of Aβ can be used as the capture substance capable of binding to the C-terminal region of the polypeptide. An anti-Aβ monoclonal antibody capable of binding to the C-terminal region of Aβ is generally available. For example, antibodies against clones H31L21 (epitope: 36 to 42), G2-11 (epitope: 33 to 42), 16C11 (epitope: 33 to 42), 21F12(epitope :34 to 42) and the like are commercially available. Among these, H31L21 is particularly preferred.

In the present embodiment as defined in the appended claims, it is preferred that a substance for detection having a labeling substance and capable of binding to the polypeptide is further brought into contact with the polypeptide to form a complex containing the polypeptide, the capture substance and the substance for detection. The term "substance for detection" as used herein refers to a substance which can bond specifically to the polypeptide that is the analyte to provide a signal that can be detected through the labeling substance. It is preferred for the substance for detection not to be immobilized onto a solid phase. Examples of the substance for detection include an antibody and an aptamer. The antibody that can be used as the substance for detection is also referred to as an "detection antibody" hereinafter. The detection antibody may be any one of a monoclonal antibody and a polyclonal antibody, and the detection antibody is preferably a monoclonal antibody.

In the present embodiment as defined in the appended claims, the substance for detection may be bonded to a C-terminal region of the polypeptide or a N-terminal region of the polypeptide. Preferably, the substance for detection has a labeling substance and can bind to a N-terminal region of the polypeptide. The N-terminal region of a polypeptide does not have a critical boundary, and may be, for example, a region lying between an amino acid residue located at the N-terminal in the amino acid sequence for the monomeric polypeptide and an amino acid residue located at a position in the vicinity of the center in the amino acid sequence. For example, when the polypeptide is composed of 50 amino acid residue, the N-terminal region may be a region lying between position-1 and an amino acid residue located at position-25. Alternatively, when a specific N-terminal region of the polypeptide is defined by the structure, physical property, function or the like thereof in the art, it is possible to follow the definition.

In the present embodiment as defined in the appended claims, the substance for detection can be produced by labeling a substance capable of recognizing an epitope present in the polypeptide (preferably a N-terminal region thereof) or the three-dimensional structure of the polypeptide (preferably a N-terminal region thereof) to bind specifically to the polypeptide with a known labeling substance that has been used conventionally in immunological techniques. Hereinafter, the epitope or the three-dimensional structure recognized by the substance for detection is also referred to as a "recognition site of the substance for detection". For example, in the case where the polypeptide is Aβ (particularly Aβ₁₋₄₂), an antibody capable of recognizing an epitope present in a region lying between an amino acid residue located at the N-terminal and an amino acid residue located at position-28, preferably position-21, more preferably position-16, in Aβ can be used as the substance for detection. An anti-Aβ monoclonal antibody capable of binding to the N-terminal region of Aβ is generally available. For example, antibodies against clones 82E1 (epitope: 1 to 16), 6E10 (epitope: 3 to 8), WO-2 (epitope: 4 to 10) and 2H4 (epitope:1 to 8) are commercially available. Among these, 82E1 is particularly preferred.

The method for labeling the antibody or the aptamer with a labeling substance is known in the art, and the method can be selected appropriately depending on the type of the labeling substance to be used. For example, the antibody or the aptamer can be bonded or linked to the labeling substance using a proper cross-linking agent, a commercially available labeling kit or the like.

The labeling substance is not particularly limited, as long as the labeling substance can generate a detectable signal directly or indirectly. Examples of the labeling substance include an enzyme, a fluorescent substance and a radioactive isotope. Examples of the enzyme include alkaline phosphatase, β-galactosidase, peroxidase, glucose oxidase, tyrosinase, acid phosphatase and luciferase. Examples of the fluorescent substance include: a fluorescent dye such as fluorescein isothiocyanate (FITC), rhodamine and Alexa Fluor (registered tradename); and a fluorescent protein such as green fluorescent protein (GFP). Examples of the radioactive isotope include ¹²⁵I, ¹⁴C and ³²P.

The timing of adding the substance for detection can be determined depending on, for example, whether or not a recognition site of the substance for detection and a recognition site of the capture substance are overlapped with each other. The wording "recognition sites are overlapped with each other" as used herein refers to the matter that recognition sites of substances capable of bonding specifically to the polypeptide are the identical to each other or partially coincide with each other. In the case where the recognition site of the substance for detection and the recognition site of the capture substance are not overlapped with each other, i.e., in the case where both of the substance for detection and the capture substance can bind to the monomeric polypeptide, the substance for detection may be added in the complex formation step, or the substance for detection may be added during the below-mentioned step of immobilizing onto the first (or second) solid phase or the below-mentioned step of collecting the first solid phase. In the case where the recognition site of the substance for detection and the recognition site of the capture substance are overlapped with each other, i.e., in the case where only one of the substance for detection and the capture substance can bind to the monomeric polypeptide, it is preferred that the substance for detection is added in the complex formation step. In this case, finally information on a polypeptide that is a multimer higher than a dimer can be acquired. In a preferred embodiment, the substance for detection is added in the complex formation step.

The complex formation step can be carried out by, for example, mixing a sample containing the polypeptide with a solution containing the capture substance. As the result of the mixing, the polypeptide is contacted with and bonded to the capture substance. In this manner, a complex containing the "(polypeptide)-(capture substance)" can be formed. In the case where the substance for detection is added in the complex formation step, a sample containing the polypeptide, a solution containing the capture substance and a solution containing the substance for detection are mixed together. As the result of the mixing, the substance for detection, the polypeptide and the capture substance are contacted with one another and are bonded together. In this manner, a sandwich complex containing the "(substance for detection)-(polypeptide)-(capture substance)" is formed. The order of the mixing of the polypeptide, the capture substance and the substance for detection is not particularly limited, and it is preferred that these components are mixed substantially simultaneously. The reaction temperature and the reaction time to be employed in the complex formation step are not particularly limited. In general, a reaction solution is allowed to leave or stirred gently at a temperature of 20 to 45°C for 15 minutes to 1 hour.

In an embodiment as defined in the appended claims in which two types of capture substances are used, a recognition site of a first capture substance and a recognition site of a second capture substance may be or may not be overlapped with each other. In the case where the recognition site of the first capture substance and the recognition site of the second capture substance are overlapped with each other, only one of the first capture substance and the second capture substance can bind to the monomeric polypeptide. In this case, finally information on a polypeptide that is a multimer higher than a dimer can be acquired.

### (Step of immobilizing onto first solid phase)

Referring to Fig. 1, as shown in step S2, subsequent to the formation of the complex, the capture substance in the complex is bonded to the first solid phase to immobilize the complex onto the first solid phase. In general, the immobilization of the complex onto the first solid phase can be carried out in a solution.

The first solid phase can be selected from known solid phases that have been used conventionally in immunological procedures. The material to be used for the solid phase can be selected from an organic polymeric compound, an inorganic compound, a biological polymer and the like. Examples of the organic polymeric compound include a latex, a rubber, polyethylene, polypropylene, polystyrene, a styrene-butadiene copolymer, poly(vinyl chloride), poly(vinyl acetate), polyacrylamide, polymethacrylate, a styrene-methacrylate copolymer, poly(glycidyl methacrylate), an acrolein-(ethylene glycol dimethacrylate) copolymer, a poly(vinylidene difluoride) (PVDF) and a silicone. Examples of the inorganic compound include a magnetic material (e.g., iron oxide, chromium oxide, cobalt, nickel, ferrite, magnetite), a glass, silica and alumina. Examples of the biological polymer include insoluble agarose, insoluble dextran, gelatin and cellulose. It is possible to use a combination of two or more of these materials. In the present embodiment, the first solid phase is preferably an insoluble particle such as a magnetic particle and a latex particle, particularly preferably a magnetic particle.

The bonding between the capture substance and the first solid phase in the complex is only needed to be a dissociable bonding. In a preferred embodiment, the capture substance and the first solid phase in the complex are bonded to each other indirectly through another substance. As the substance, a combination of two substances that can be bonded specifically to each other and are dissociable is preferred. Hereinafter, the two substances are called as a "bonding substance" and a "bonding partner", respectively. The combination of the bonding substance and the bonding partner is known in the art, and examples of the combination include a combination of an antigen (excluding the analyte) and an antibody thereof, a combination of a ligand and a receptor thereof, a combination of an oligonucleotide and a strand complementary thereto, a combination of a biotin-type compound (including biotin and a biotin analogue such as desthiobiotin) and an avidin-type compound (including avidin and an avidin analogue such as streptavidin), a combination of nickel and a histidine tag, and a combination of glutathione and glutathione-S-transferase. Preferred examples of the combination of an antigen and an antibody thereof include a combination of a hapten and an anti-hapten antibody, and a combination of biotin (or desthiobiotin) and an anti-biotin antibody (or an anti-desthiobiotin antibody). A particularly preferred example of the combination of a hapten and an anti-hapten antibody is a combination of 2,4-dinitrophenyl (DNP) group and an anti-DNP antibody.

In an exemplary embodiment in which a single type of capture substance is used, it is preferred that the capture substance has both of the first bonding substance and the second bonding substance and can bind to a C-terminal region of the polypeptide. In the embodiment as defined in the appended claims in which two types of capture substances are used, it is that the capture substance includes a first capture substance having the first bonding substance and a second capture substance having the second bonding substance and at least one of the first capture substance and the second capture substance can bind to a C-terminal region of the polypeptide. In both of the embodiments, it is preferred that the first solid phase has a first bonding partner capable of bonding specifically to a first bonding substance and the below-mentioned second solid phase has a second bonding partner capable of bonding specifically to a second bonding substance.

In the present embodiment as defined in the appended claims, the capture substance and the first solid phase in the complex bind to each other through a specific bonding between the first bonding substance and the first bonding partner. As a result, the complex is immobilized onto the first solid phase. In a preferred embodiment, the first bonding substance is a DNP group and the first bonding partner is an anti-DNP antibody. The method for bonding the substance to the capture substance and solid phase is known in the art. For example, in the case where biotin or DNP is bonded to an antibody, a method is known in which a cross-linking agent capable of reacting with an amino group or a thiol group in the antibody (e.g., maleimide, N-hydroxysuccinimide) is used. Alternatively, a commercially available labeling kit may be used. As the method for bonding the bonding substance or the bonding partner to a solid phase, a physical adsorption method, a covalent bonding method, an ionic bonding method and the like are known.

The immobilization step can be carried out by bringing the complex into contact with the first solid phase. For example, in the case where the first solid phase has a particulate form, a liquid containing the complex is mixed with the first solid phase to cause the contact between the complex and the first solid phase. In the case where the substance for detection is added in the immobilization step, the complex, the substance for detection and the first solid phase are brought into contact with one another. The reaction temperature and the reaction time are not particularly limited. In general, the reaction solution is allowed to leave or stirred gently at a temperature of 20 to 45°C for 15 minutes to 3 hours.

### (Step of collecting first solid phase)

Referring to Fig. 1, as shown in step S3, in the method according to the present embodiment, the first solid phase having the complex immobilized thereon is collected. The wording "collecting a solid phase" is not limited to the collection of only a solid phase having the complex immobilized thereon, and the wording also includes the collection of a solid phase on which the complex is immobilized in such a state where other substance is also contained in a trace amount. In the reaction system, in addition to the first solid phase having the complex immobilized thereon, an unreacted component such as contaminants contained in the sample and a surplus of the antibody is present. The term "unreacted component" as used herein refers to a component which is different from the complex immobilized on the solid phase and is not bonded to the solid phase and is therefore in a free form. In the collection step, the first solid phase having the complex immobilized thereon is separated from the unreacted component and is collected. Therefore, by carrying out the collection step, an unreacted component that adversely affects the below-mentioned information acquisition step can be removed. The collection step of this type is commonly called as a "B/F separation". In the collection step, it is not needed to remove the unreacted component completely. The unreacted component may be allowed to remain, as long as the unreacted component does not adversely affect measurements.

The method for collecting the first solid phase having the complex immobilized thereon is known in the art, and the method can be selected appropriately depending on the type of the first solid phase used. For example, in the case where a magnetic particle is used, the magnetic particle can be collected by magnetic separation. More specifically, a magnet is brought close to the wall surface of a container containing a magnetic particle each the complex immobilized thereto to immobilize the magnetic particle onto the wall surface of the container, and then a liquid is removed by sucking to collect the magnetic particle having the complex immobilized thereon. In the case where an insoluble particle such as a latex particle is used, the particle is precipitated by centrifugation and then a liquid is removed by sucking to collect the insoluble particle having the complex immobilized thereon.

In the present embodiment, the step of washing the collected first solid phase can be further included. The washing of the first solid phase can be carried out by, for example, adding a wash solution to the collected first solid phase and then removing the wash solution from the first solid phase. As the wash solution, a buffer solution that cannot impair the complex immobilized on the first solid phase is preferred. As the wash solution of this type, a buffer solution containing a surfactant is particularly preferred, and examples of the buffer solution include TBST (a Tris-buffered saline containing 0.05% of Tween20) and PBST (a phosphate-buffered saline containing 0.05% of Tween20). A commercially available wash solution, such as HISCL wash solution (Sysmex Corporation), can also be used. By the washing, a component that is adsorbed non-specifically onto the first solid phase or the complex can be removed.

### (Step of immobilizing onto second solid phase)

Referring to Fig. 1, as shown in step S4, in the method according to the present embodiment, the complex is released from the collected first solid phase. The capture substance in the released complex is bonded to the second solid phase to immobilize the complex onto the second solid phase. The complex immobilized on the second solid phase is subjected to a specific measurement system in the below-mentioned information acquisition step to acquire information on the polypeptide.

The method for releasing the complex on the solid phase is known in the art. For example, a method can be mentioned, in which a substance capable of dissociating the bonding between the capture substance in the complex and the first solid phase (also referred to as a "releasing agent", hereinafter). The releasing agent is known in the art, and the releasing agent can be selected appropriately depending on the type of the bonding between the capture substance and the first solid phase. For example, in the case where the capture substance in the complex and the first solid phase are bonded to each other through physical adsorption, the complex can be released from the first solid phase by using a solution containing a surfactant as the releasing agent. In the case where the capture substance in the complex and the first solid phase are bonded to each other through ionic bonding, the complex can be released from the first solid phase by using a solution containing an ion.

In the case where the capture substance and the first solid phase are bonded indirectly through specific bonding between the first bonding substance and the first bonding partner, the complex can be released by using a releasing agent capable of dissociating the bonding between the first bonding substance and the first bonding partner. The releasing agent is also known in the art, and the releasing agent can be selected appropriately depending on the combination of the bonding substance and the bonding partner. For example, in the case of the bonding between a hapten and an anti-hapten antibody, the hapten or a derivative thereof can be used as the releasing agent. For example, in the case of the bonding between a DNP group and an anti-DNP antibody, a dinitrophenyl amino acid can be used as the releasing agent. In the case of the bonding between biotin (or desthiobiotin) and avidin (or streptavidin), biotin can be used as the releasing agent.

In the case where the complex is released using the releasing agent, the treatment temperature and the treatment time can be preset appropriately depending on the type of the releasing agent to be used. In general, the reaction solution may be allowed to leave or stirred gently at a temperature of 20 to 45°C for 3 to 15 minutes after the addition of the releasing agent.

After the addition of the releasing agent, it is preferred that the complex released from the first solid phase and the first solid phase are separated from each other, and a liquid containing the released complex is collected. For example, in the case where a particle is used as the first solid phase, the complex is released from the first solid phase, and then the first solid phase is collected on the wall surface or the bottom of a container by a magnetic force or centrifugation in the same manner as mentioned with respect to the collection step. Subsequently, a liquid containing the complex is collected.

The immobilization of the complex onto the second solid phase can be carried out by, for example, bringing the complex and the second solid phase into contact with each other. For example, in the case where the second solid phase has a particulate form, a liquid containing the complex is mixed with the second solid phase to cause the complex and the second solid phase to contact with each other. In the case where the second solid phase has a thin-plate-like form, a liquid containing the complex is dropped onto the second solid phase to cause the complex and the second solid phase to contact with each other. The reaction temperature and the reaction time are not particularly limited. In general, the reaction solution is allowed to leave or stirred gently at a temperature of 20 to 45°C for 15 minutes to 3 hours.

As mentioned above, when the complex released from the first solid phase is brought into contact with the second solid phase that is different from the second solid phase, the capture substance in the complex is bonded to the second solid phase to cause the complex to be transferred onto the second solid. The term "second phase that is different from the first solid phase" as used herein refers to a novel solid phase which is different from the first solid phase that is present from the point of time at which the complex is added in the step of immobilizing onto the first solid phase. Namely, in the step of immobilizing the complex onto the second solid phase, it is not intended to bond the complex released from the first solid phase to the first solid phase again. In a preferred embodiment, a liquid containing the complex released from the first solid phase is collected, and the collected liquid is brought into contact with a newly provided second solid phase.

The material and the form of the second solid phase are the same as those materials and forms mentioned above with respect to the first solid phase. The material for the second solid phase may be the same as or different from the material for the first solid phase. Examples of the form of the second solid phase include a particle, a thin film, a membrane, a microtiter plate, a micro tube and a test tube. The material and the form of the solid phase may be selected appropriately depending on the type of the measurement means to be employed. For example, in the case where the polypeptide is observed with a microscope, a solid phase made from a material through which light can pass and a thin-plate-like form (e.g., a glass slide) is preferred.

The mode of the binding between the capture substance in the complex and the second solid phase is not particularly limited. For example, the capture substance and the second solid phase may be bonded to each other directly through physical adsorption, ionic bonding or the like. Alternatively, the capture substance and the second solid phase may be bonded to each other indirectly through another substance. As the substance, a combination of the bonding substance and the bonding partner can be mentioned. The combination of the second bonding substance and the second bonding partner is preferably different from the combination of the first bonding substance and the first bonding partner. In the case where a single type of capture substance is used, a capture substance having a biotin-type compound and a DNP group, a first solid phase having an anti-DNP antibody immobilized on the surface thereof and a second solid surface having an avidin-type compound immobilized on the surface thereof can be used, for example. In the case where two types of capture substances are used, a first capture substance having a DNP group, a second capture substance having a biotin-type compound, a first solid phase having an anti-DNP antibody immobilized on the surface thereof and a second solid phase having an avidin-type compound immobilized on the surface thereof can be used, for example.

In a preferred embodiment, the step of washing the first solid phase having the complex immobilized thereon is further included. The washing of the first solid phase can be carried out in the same manner as mentioned with respect to the washing of the second solid phase.

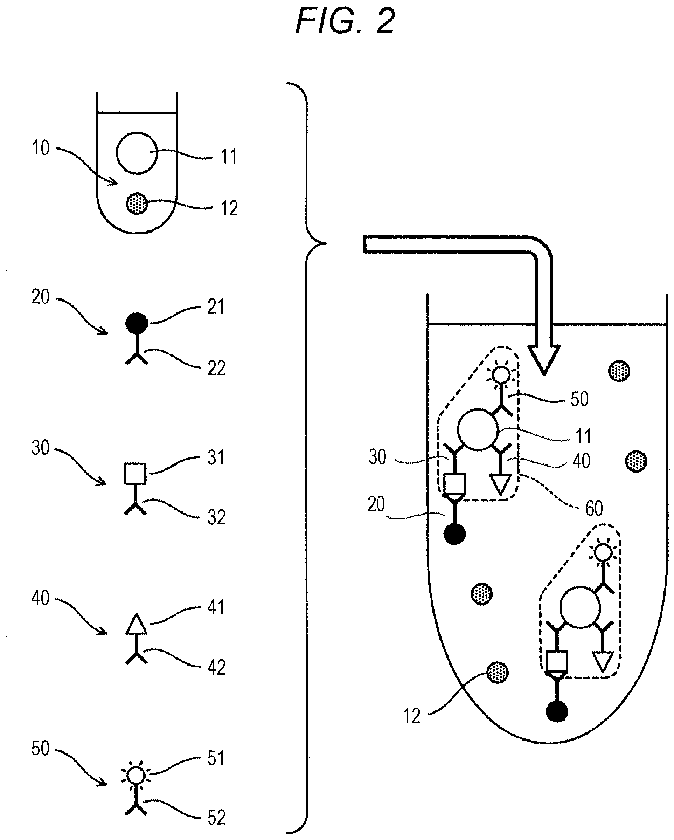

Hereinbelow, the process from the step of forming the complex to the step of immobilizing the complex onto the second solid phase will be described with reference to drawings. These drawings only illustrate one example of the present embodiment, and is not intended to limit the present disclosure. As shown in Fig. 2, a sample 10 contains a polypeptide that is an analyte 11 and a contaminant 12. The contaminant 12 is an undesired substance that is different from the polypeptide 11, and is, for example, a protein other than the polypeptide 11. In Fig. 2, the sample 10 containing the polypeptide 11 and the contaminant 12, a first solid phase 20, a capture substance 30 having a first bonding substance 31, a capture substance 40 having a second bonding substance 41, and a substance for detection 50 having a fluorescent substance 51 are mixed together to form a complex 60. In Fig. 2, each of the capture substance and the substance for detection is an antibody. Each of the antibody 32 and the antibody 42 is an antibody capable of binding to a C-terminal region of the polypeptide 11, and an antibody 52 is an antibody capable of binding to a N-terminal region of the polypeptide 11. The first solid phase 20 is a magnetic particle 21 having a first bonding partner 22 immobilized on the surface thereof. In Fig. 2, the first bonding substance 31 is a DNP group, the second bonding substance 41 is biotin, and the first bonding partner 22 is an anti-DNP antibody. The first capture antibody 30 in the complex 60 and the first solid phase 20 are bonded to each other through specific bonding between the first bonding substance 31 and the first bonding partner 22. In this manner, the complex 60 is immobilized onto the first solid phase 20.

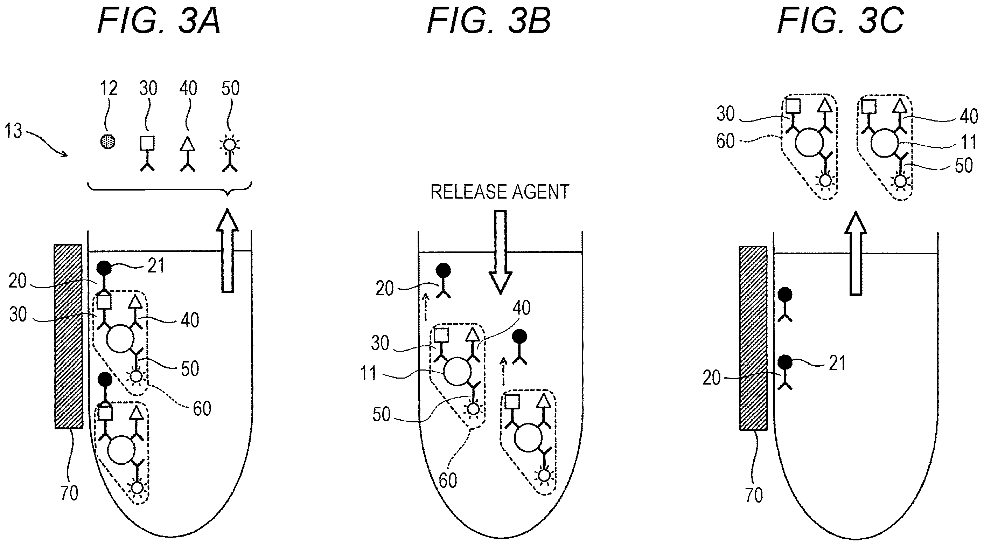

Referring to Fig. 3A, the complex 60 immobilized on the first solid phase 20 is separated from an unreacted component 13. In Fig. 3A, the unreacted component 13 includes a contaminant 12, and also includes the capture substance 30, the capture substance 40 and the substance for detection 50 that are not involved in the formation of the complex. When a magnet 70 is moved close to a container, a magnetic particle 21 is attracted to an inner wall of the container. At this time, the complex 60 is immobilized on the first solid phase 20, and therefore the complex 60 is also attracted to the inner wall of the container together with the magnetic particle 21. When the liquid in the container is removed in this state, the complex 60 is separated from the unreacted component 13.

Referring to Fig. 3B, the bonding between the first bonding substance 31 and the first bonding partner 22 is dissociated by adding dinitrophenyl lysine as a releasing agent. As a result, the first solid phase is dissociated from the complex 60. Referring to Fig. 3C, the complex 60 released from the first solid phase 20 is separated from the first solid phase 20. When a magnet 70 is moved close to a container, a magnetic particle 21 is attracted to an inner wall of the container. A liquid in the container is collected in this state, thereby collecting the complex 60 selectively.

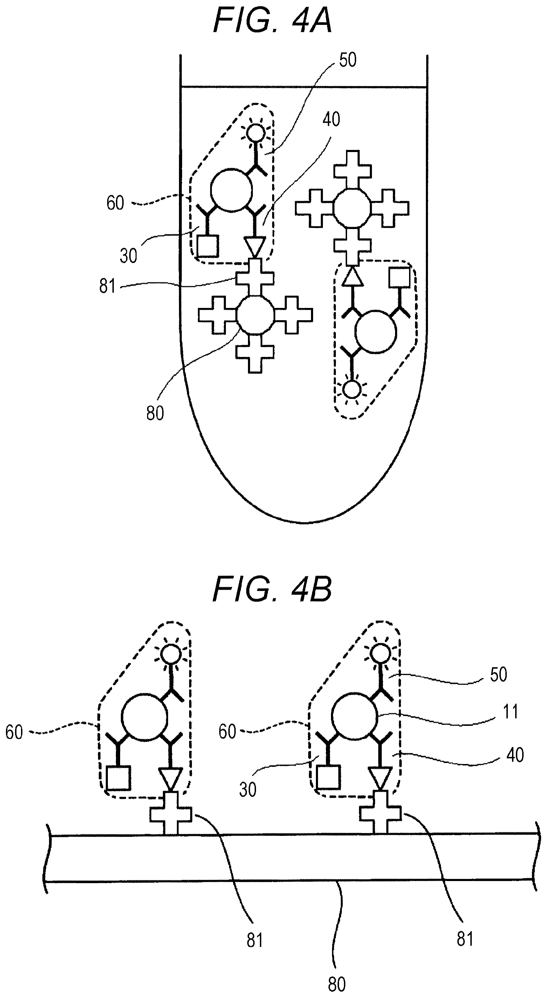

In Fig. 4A, an example in which an insoluble particle is used as the second solid phase is shown. Referring to Fig. 4A, a liquid containing a complex 60 is mixed with a second solid phase 80 having a second bonding partner 81, thereby immobilizing the complex 60 onto the second solid phase 80. The second bonding partner 81 is an avidin-type compound. A second capture antibody 40 in the complex 60 is bound to the second solid phase 80 through specific bonding between the second bonding substance 41 and the second bonding partner 81. In this manner, the complex 60 can be immobilized onto the second solid phase 80. Alternatively, as shown in Fig. 4B, a thin-film-like solid phase may be used as the second solid phase. Referring to Fig. 4B, a liquid containing the complex 60 is dropped onto a first solid phase 80 containing a second bonding partner 81, thereby immobilizing the complex 60 onto the second solid phase 80.

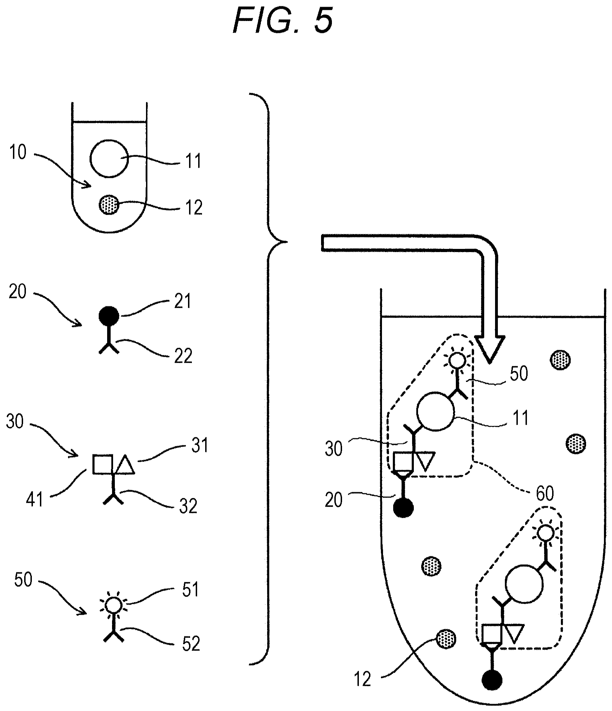

In the complex formation step shown in Fig. 2, as two types of capture substances each capable of binding to a C-terminal region of the polypeptide 11, a capture substance 30 having a first bonding substance 31 and a capture substance 40 having a second bonding substance 41 are used. Alternatively, as shown in Fig. 5, a capture substance 30 having both of a first bonding substance 31 and a second bonding substance 41 may be used in place of these two types of capture substances. In Fig. 5, a sample 10 containing a polypeptide 11 and a contaminant 12, a first solid phase 20, a capture substance 30 having both of a first bonding substance 31 and a second bonding substance 41, and a substance for detection 50 having a fluorescent substance 51 are mixed together to form a complex 60. The capture substance 30 in the complex 60 and the first solid phase 20 are bonded through specific bonding between the first bonding substance 31 and the first bonding partner 22. In this manner, the complex 60 is immobilized onto the first solid phase 20. After the formation of the complex 60, the complex 60 is collected selectively by magnetic separation, like Fig. 3.

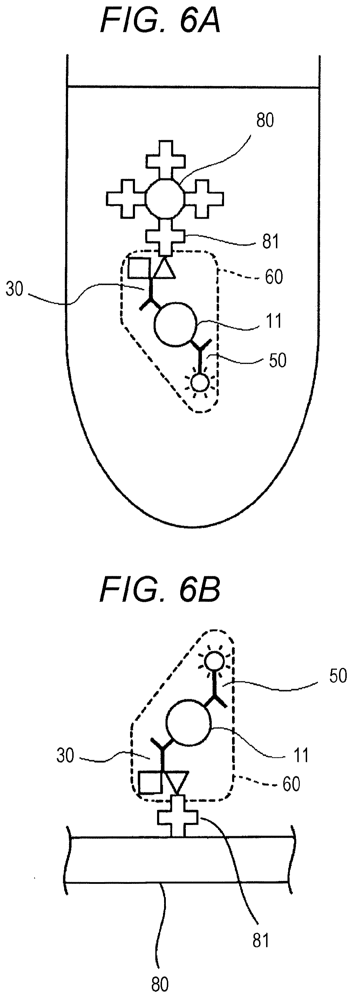

Referring to Fig. 6A, a liquid containing a complex 60 is mixed with a second solid phase 80 containing a second bonding partner, thereby immobilizing the complex 60 onto the second solid phase 80 through specific bonding between the second bonding substance 41 and the second bonding partner 81. Referring to Fig. 6B, a liquid containing a complex 60 is dropped onto a second solid phase 80 containing a second bonding partner 81, thereby immobilizing the complex 60 onto the second solid phase 80.

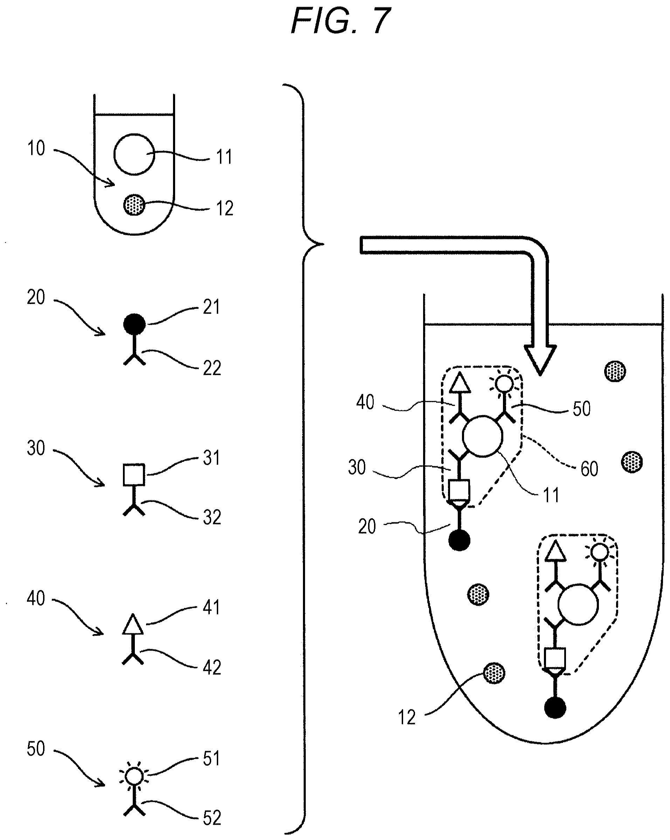

Referring to Fig. 7, an example in which a capture substance capable of bonding to a C-terminal region of the polypeptide and a capture substance capable of binding to a N-terminal region of the polypeptide are used is described. As shown in Fig. 7, a sample 10 contains a polypeptide that is an analyte 11 and a contaminant 12. In Fig. 7, the sample 10 containing the polypeptide 11 and the contaminant 12, a first solid phase 20, a capture substance 30 having a first bonding substance 31, a capture substance 40 having a second bonding substance 41, and a substance for detection 50 having a fluorescent substance 51 are mixed together to form a complex 60. In Fig. 7, the capture substance 30 (an antibody 32) is an antibody capable of binding to a C-terminal region of the polypeptide 11, and each of the capture substance 40 (an antibody 42) and the substance for detection 50 (an antibody 52) is an antibody capable of binding to a N-terminal region of the polypeptide 11. The first solid phase 20 is a magnetic particle 21 having a first bonding partner 22 immobilized on the surface thereof. In Fig. 7, the first bonding substance 31 is a DNP group, the second bonding substance 41 is biotin, and the first bonding partner 22 is an anti-DNP antibody. The capture substance 30 in the complex 60 and the first solid phase 20 are bonded through specific bonding between the first bonding substance 31 and the first bonding partner 22. In this manner, the complex 60 is immobilized onto the first solid phase 20.

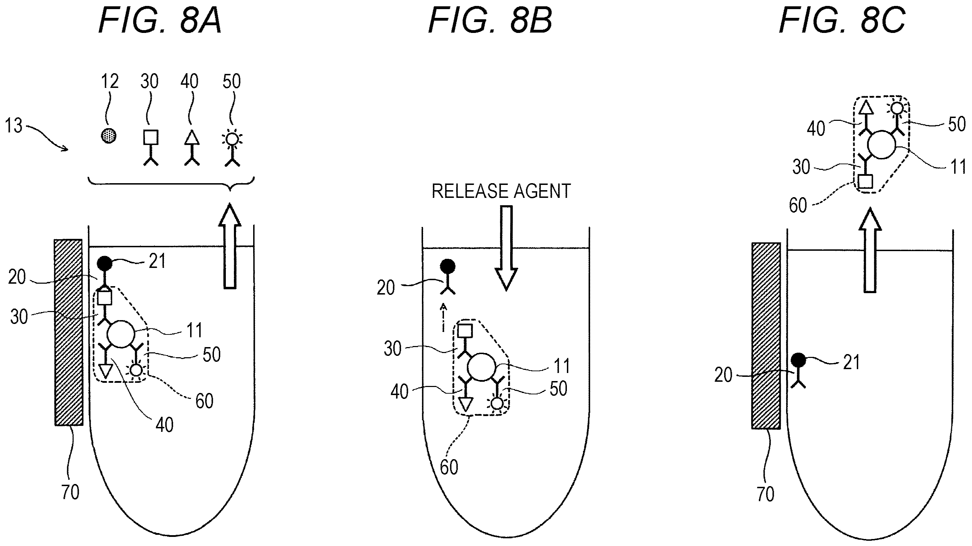

In Fig. 8A, a complex 60 immobilized on a first solid phase 20 is collected by magnetic separation. In Fig. 8B, the bonding between a first bonding substance 31 and a first bonding partner 22 is dissociated by using the by dinitrophenyl lysine as the releasing agent. As a result, the first solid phase is dissociated from the complex 60. In Fig. 8C, the complex released from the first solid phase 20 is magnetically separated from the first solid phase 20 to collect the complex 60 selectively. Details about the steps shown in Fig. 8 are similar to those mentioned with respect to Fig. 3.

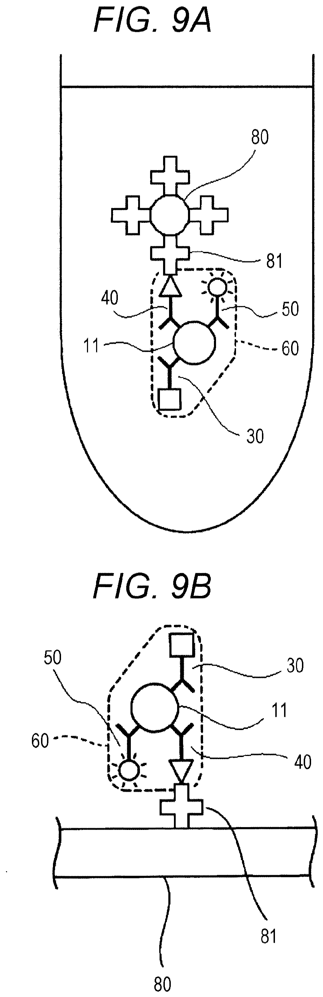

In Fig. 9A, a liquid containing a complex 60 and a second solid phase 80 containing a second bonding partner 81 are mixed together, thereby immobilizing the complex 60 onto the second solid phase 80 through specific bonding between the second bonding substance 41 and the second bonding partner 81. In Fig. 9B, a liquid containing a complex 60 is dropped onto a second solid phase 80 containing a second bonding partner 81, thereby immobilizing the complex 60 on the second solid phase 80.

In the present embodiment, each step or all steps in a sequence of steps from the step of forming the complex to the step of releasing and collecting the complex from the first solid phase may be carried out by a manual procedure or using a device. Alternatively, each step or all steps in a sequence of steps from the formation of the complex to the immobilization of the complex onto the second solid phase may be carried out by a manual procedure or using a device. Examples of the device include an automatic sample treatment device, an automatic immunoassay device and a device for manufacturing a glass slide for microscopic observation use.

### (Information acquisition step)

Referring to Fig. 1, as shown in step S5, in the method according to the present embodiment, information on the polypeptide is acquired from the complex immobilized on the second solid phase. The information on the polypeptide may be information on the quantity of the polypeptide or information on the structure of the polypeptide.

The information on the quantity of the polypeptide may be quantitative information or quantitative information. An example of the quantitative information is the presence or absence of the polypeptide. Examples of the quantitative information include the concentration of the polypeptide, the content (weight) of the polypeptide, and a measurement value thereof. The quantitative information also includes semiquantitative information which indicates the quantity of the polypeptide in a graded manner, such as "a small quantity", "a medium quantity" and "a large quantity".

Examples of the information on the structure of the polypeptide include information on the size of the polypeptide, information on the morphology of the polypeptide, and information on the aggregation degree of the polypeptide. In a preferred embodiment, information on the structure of the polypeptide is acquired by imaging the polypeptide on the second solid phase with a microscope to obtain an image of the polypeptide. The type of the microscope is not particularly limited, as long as an image of the peptide can be obtained. Examples of the microscope include a fluorescence microscope, a super-resolving microscope, a Raman microscope, a probe microscope and an electron microscope.

In the present embodiment, information on the polypeptide is acquired by measuring a signal coming from the labeling substance in the complex immobilized on the second solid phase. In the case where information on the quantity of the polypeptide is to be acquired, it is preferred to measure the intensity of the signal or the like in terms of a numerical value. For example, information on the concentration or content of a polypeptide can be acquired by assigning an obtained measurement value to a calibration curve produced from measurement values for a polypeptide having a known concentration. In the case where information on the structure of the polypeptide is to be acquired, it is preferred to obtain an image associated with the signal.

The method for measuring a signal coming from a labeling substance is known in the art. In the present embodiment, a proper method can be selected depending on the signal coming from the labeling substance. For example, in the case where the labeling substance is an enzyme, it is possible that the enzyme in the complex is reacted with a substrate for the enzyme and then a signal, e.g., light, color, generated from a reaction produce produced as the result of the enzymatic reaction can be measured using a known device. Examples of the device include a spectrophotometer and a luminometer.

The substrate for an enzyme can be selected appropriately from known substrates depending on the type of the enzyme to be used. For example, in the case where alkaline phosphatase is used as the enzyme, examples of the substrate for the enzyme include: a chemiluminescent substrate such as CDP-Star (registered tradename) (disodium 4-chloro-3-(methoxyspiro[1,2-dioxetane-3,2'-(5'-chloro)tricyclo[3. 3. 1. 13, 7]decan]-4-yl)phenylphosphate) and CSPD (registered tradename) (disodium 3-(4-methoxyspiro[1,2-dioxetane-3,2-(5'-chloro)tricyclo[3.3.1.13,7]decan]-4-yl)phenylphosphate); a luminescent substrate such as p-nitrophenyl phosphate, a 5-bromo-4-chloro-3-indolyl phosphate (BCIP), 4-nitro blue tetrazolium chloride (NBT) and iodonitrotetrazolium (INT); a fluorescent substrate such as 4-methylumbelliferylphosphate (4MUP); and a chromogenic substrate such as 5-bromo-4-chloro-3-indolyl phosphate (BCIP), disodium 5-bromo-6-chloro-indolyl phosphate and p-nitrophenylphosphate. In the case where β-galactosidase is used as the enzyme, the substrate for the enzyme is, for example, 4-methylumbelliferyl-β-D-galactopyranoside.

In the case where the labeling substance is a fluorescent substance, it is possible that the complex is irradiated with excitation light, and then fluorescence generated from the fluorescent substance in the complex is measured using a known device such as a fluorescence microplate reader. In the case where the labeling substance is a radioactive isotope, radioactive ray generated from an radioactive isotope in the complex can be measured using a known device such as a scintillation counter.

Hereinbelow, the case where information on the structure of the polypeptide is acquired using a super-resolving fluorescence microscope that is one type of super-resolving microscope will be described with reference to drawings. A super-resolving microscope is a microscope having resolution performance beyond the limit of diffraction of light.

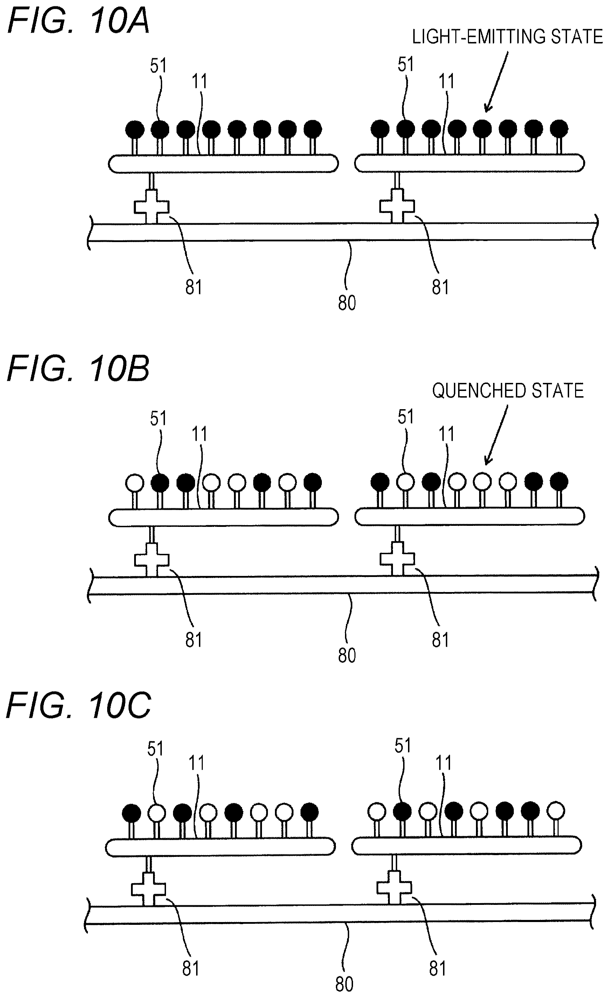

In Fig. 10, a complex immobilized on a thin-film-like second solid phase 80 (also referred to as a "substrate 80", hereinafter). In the complex, a detection antibody labeled with a fluorescent substance 51 is bonded to a polypeptide 11. The fluorescent dye 51 is so configured that the state of the fluorescent dye 51 can be switched between a light-emitting state where fluorescent light is emitted and a quenched state where fluorescent light is not emitted when the fluorescent dye 51 is irradiated with excitation light sustainably. This optically switched fluorescent dye is commercially available from, for example, Molecular Probes Inc. In Fig. 10, the fluorescent dye 51 that is in a light-emitting state is indicated by a black circle, and the fluorescent dye 51 that is in a quenched state is indicated by a white circle.

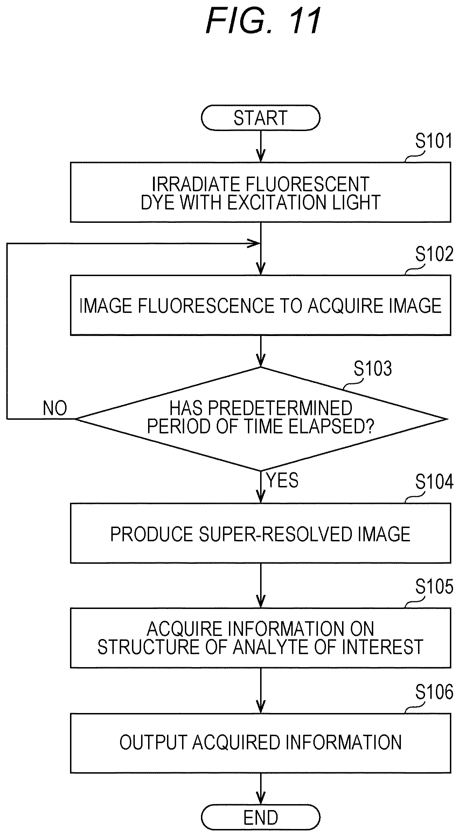

Referring to Fig. 11, in step S101, excitation light is emitted to fluorescent dye 51 on the substrate 80. As shown in Fig. 10A, in the initial state, all molecules of the fluorescent dye 51 are in the light-emitting state. Upon the start of the irradiation with the excitation light, fluorescent light is excited from all molecules of the fluorescent dye 51. Subsequently, when the fluorescent dye 51 is irradiated with the excitation light sustainably, the distribution of the fluorescent dye 51 in the light-emitting state is changed with the elapse of time, as shown in, for example, Figs. 10B and 10C.

In step S102, during the irradiation of the fluorescent dye 51 with the excitation light, generated fluorescent light is imaged to acquire an image of the fluorescent dye 51. In step S102, the imaging is repeated during the irradiation of the fluorescent dye 51 with the excitation light. In this manner, 3000 images, for example, can be acquired. Since the distribution of the fluorescent dye 51 in the light-emitting state is changed with elapse of time, the distributions of fluorescent light on the acquired images are different among the acquired images.

In step S103, a predetermined period of time is elapsed, and it is determined as to whether or not the acquisition of a desired image is completed. Until the predetermined period of time is elapsed, the imaging is repeated in step S102. When the predetermined period of time is elapsed and the acquisition of the desired image is completed, the procured proceeds to step S104. In this manner, the image is acquired, it becomes possible to acquire information on the structure of the polypeptide 11 in the subsequent step.

Alternatively, an image may also be acquired by employing a step based on a technique such as STORM (Stochastic optical reconstruction microscopy), PALM (Photoactivated localization microscopy), STED (Stimulated emission depletion) or SIM (Structured illumination microscopy) in place of step S101 to S103. In the case where the image is acquired by a step based on STORM, the fluorescent dye 51 is so configured that the state of the fluorescent dye 51 can be switched between an active state where fluorescent light is generated and an inactive state where fluorescent light is not generated. By switching the state of the fluorescent dye 51 The distribution of the fluorescent light between the active state and the inactive state with two types of light, a plurality of images having different fluorescent light distributions from each other can be acquired.

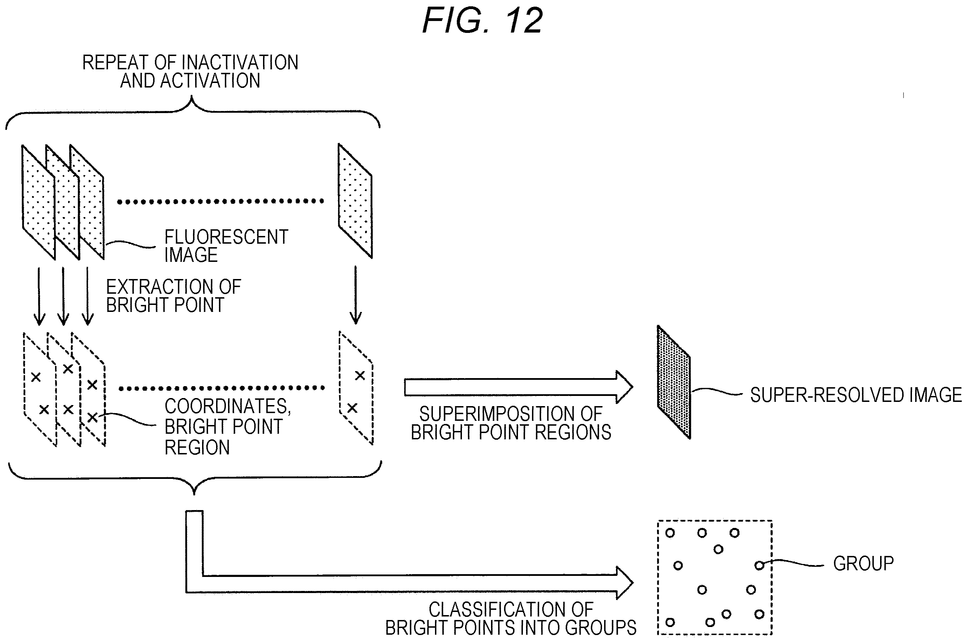

Subsequently, in step S104, a super-resolved image is produced. As shown in Fig. 12, the super-resolved image is produced on the basis of the plurality of fluorescent images acquired in step S102 shown in Fig. 11. Point spread function (PSF) fitting on the image system is carried out with respect to each of the fluorescent images, and bright points of the fluorescent light are extracted. More specifically, bright points of the fluorescent light are extracted on the basis of gaussian fitting. As a result, a coordinate of each of the bright points and an error of the fitting can be obtained in a two-dimensional plane. With respect to a bright point in a fluorescent region which coincides with a reference waveform in a given range, a bright point region having a largeness in area corresponding to the aforementioned range is assigned by the gaussian fitting. With respect to a bright point in a fluorescent region that coincides with the reference waveform only at a single point, a bright point region having a lowest level of width is assigned. In this manner, bright point regions obtained respectively from the fluorescent images are superposed on each other to produce a super-resolved image.

Accordingly, in the case where 3000 fluorescent images are obtained in step S102, the super-resolved image of the fluorescent images is produced by extracting bright points from the 3000 fluorescent images and superposing the bright point regions of the extracted bright points on each other.

Now taking a look back over Fig. 11, in step S105, information of the structure of the polypeptide 11 is acquired. In step S105, as the information on the structure of the polypeptide 11, the size, morphology, structure, aggregation degree or the like of the polypeptide 11 can be acquired.

In step S105, the information on the structure of the polypeptide 11 is acquired by the following procedure. As shown in Fig. 12, bright points extracted in the production of a super-resolved image obtained in step S104 in Fig. 11 are classified into a group corresponding to aggregated polypeptide 11. Namely, firstly, all of bright points extracted from a plurality of fluorescent image are mapped on a coordinate plane. Subsequently, the coordinate plane is scanned in a reference region having a specific largeness in area, and the number of the bright points contained in the reference area is obtained. The position of a reference area in which the number of bright points is larger than a threshold value and is also larger than a surrounding area is extracted, and the bright points contained in the reference area at the extracted position are classified into a single group. The single group thus obtained is deemed as a single cluster of aggregates of the polypeptide 11.

The method for classifying bright points into a single group is not limited to this method, and may be another clustering technique. For example, a region having a pixel value equal to or larger than a specified threshold value on a fluorescent image obtained by adding all of fluorescent images together may be deemed as a single cluster of aggregates. Alternatively, a region having a pixel value equal to or larger than a specified threshold value on a fluorescent image obtained imaging fluorescent light excited from all molecules of the fluorescent dye 51 immediately after the start of step S101 in the information acquisition step may be deemed as a single cluster of aggregates.

Subsequently, the following types of information are acquired on every aggregates of the polypeptide 11 on the basis of a super-resolved image. Namely, with respect to the size of the polypeptide 11, length in the longer length direction, a length in the shorter length direction, a periphery, an area and the like can be acquired. With respect to the morphology of the polypeptide 11, an aspect ratio, circularity, the number of branches, a branching angle and the like can be acquired. The aspect ratio can be obtained by, for example, dividing a length in the longer length direction by a length in the shorter length direction. With respect to the structure of the polypeptide 11, information on which a cluster of aggregates of the polypeptide 11 is among a primary structure, a secondary structure, a third-order structure and a four-order structure, can be obtained. With respect to the aggregation degree of the polypeptide 11, the number of monomers forming a cluster of aggregates can be obtained. The number of the monomers can be obtained by comparing between the standard size of the monomer and the size of the cluster of the aggregates.

In step S105, the information on the structure of the polypeptide 11 is acquired on the basis of the super-resolved image. However, the method for acquiring the information is not limited to this procedure, and the information may also be acquired on the basis of a fluorescent image obtained by imaging fluorescent light generated from the fluorescent dye 51. For example, the information on the structure of the polypeptide 11 may be obtained on the basis of a fluorescent image obtained by imaging fluorescent light generated from all molecules of the fluorescent dye 51 immediately after the start of step S101. In this case, however, it is impossible to analyze at resolution performance beyond the diffraction limit of light. Therefore, it is preferred to acquire the information on the structure of the polypeptide 11 on the basis of the super-resolved image, as mentioned above.

Now taking a look back over Fig. 11, in step S106, the information acquired in step S105 is output. More specifically, the acquired information is displayed on a display unit composed of a screen. Alternatively, the acquired information may be output as a sound from a speaker, or the acquired information may be transmitted as digital data to another device.

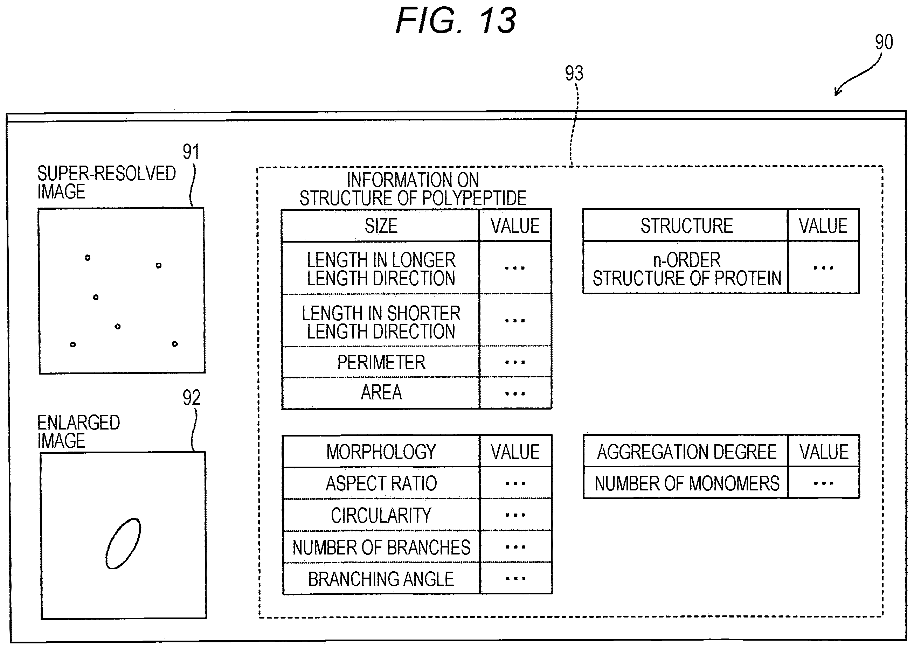

Referring to Fig. 13, a screen 90 displayed in the display unit in step S106 is described. The screen 90 is provided with images 91 and 92 and a region 93. The image 91 is the super-resolved image obtained in step S105 in Fig. 11. The image 92 is an enlarged image of a part of the image 91. The region 93 is a region on which the information on the structure of the polypeptide 11 which is acquired in step S106 in Fig. 11. When the screen 90 as shown in Fig. 13 is displayed in the information acquisition step, a physician or the like can visually know the super-resolved image and the information on the structure of the polypeptide 11. Therefore, the physical or the like can diagnose a clinical condition smoothly and can decide the strategy for the clinical condition.

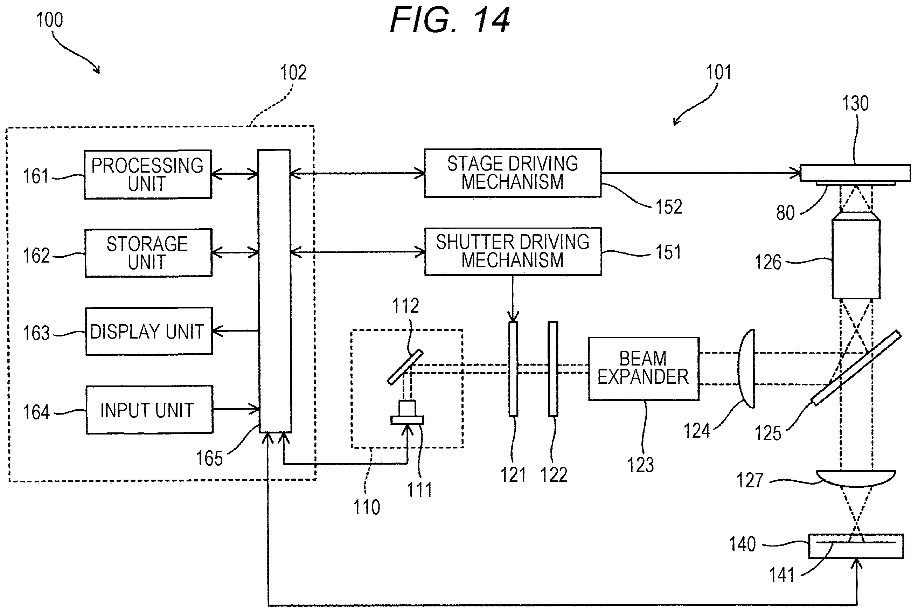

In the present embodiment, the information acquisition step may be performed automatically using a detection device 100 shown in Fig. 14. A detection device 100 is provided with an information acquisition unit 101 and an information processing unit 102. The detection device 100 is a device for performing each step in the information acquisition process shown in Fig. 11 automatically.

The information acquisition unit 101 is provided with a light source unit 110, a shutter 121, a quarter-wave retarder 122, a beam expander 123, a condenser lens 124, a dichroic mirror 125, an objective lens 126, a condenser lens 127, a stage 130, an imaging unit 140, a shutter driving mechanism 151, and a stage driving mechanism 152. On the stage 130, a substrate 80 having a polypeptide 11 immobilized thereon is mounted.

The light source unit 110 is provided with a light source 111 and a mirror 112. The light source 111 can emit excitation light. As the light source 111, a laser beam source can be used preferably, and a mercury lamp, a xenon lamp, a LED or the like may also be used. The excitation light emitted from the light source 111 can change the state of fluorescent dye 51 bonded to the polypeptide 11 into a light emitting state or a quenched state, and can excite the fluorescent dye 51 in a light emitting state to generate fluorescent light. The mirror 112 can reflect the excitation light coming from the light source 111 to guide the excitation light to the shutter 121.

In the case where the fluorescent dye 51 is so configured that the state of the fluorescent dye 51 can be switched between an active state where fluorescent light is generate and an inactive state where fluorescent light is not generated, the light source unit 110 is so configured as to have two light sources, a mirror and a dichroic mirror. In this case, one of the light sources can emit light capable of shifting the state of the fluorescent dye 51 into an active state, and the other can emit light capable of shifting the state of the fluorescent dye 51 into an inactive state. Optical axes of two types of light respectively emitted from the two light sources are made coincides with each other by means of the mirror and the dichroic mirror.

The shutter 121 can be driven by the shutter driving mechanism 151, and can be switched between a state where the excitation light emitted from the light source unit 110 can pass through the shutter 121 and a state where the excitation light emitted from the light source unit 110 can be blocked. In this manner, the time of irradiation of the analyte 11 with the excitation light can be adjusted. The shutter driving mechanism 151 is composed of, for example, a motor, a spring or the like. The quarter-wave retarder 122 can convert the excitation light having linear polarization which is emitted from the light source unit 110 to circularly polarized light. The fluorescent dye 51 can respond to excitation light having a specific polarization direction. Therefore, when the excitation light emitted from the light source unit 110 is converted to circularly polarized light, it becomes easier for the polarization direction of the excitation light to coincide with the polarization direction to which the fluorescent dye 51 responds. As a result, fluorescent light from the fluorescent dye 51 can be excited with high efficiency. The beam expander 123 can expand the irradiation region of the excitation light on the substrate 80. The condenser lens 124 can condense the excitation light in such a manner that parallel light can be emitted from the objective lens 126 toward the substrate 80.

The dichroic mirror 125 can reflect the excitation light emitted from the light source unit 110 and can permit the passage of the excitation light generated from the fluorescent dye 51 therethrough. The objective lens 126 can guide the excitation light reflected on the dichroic mirror 125 to the substrate 80. The stage 130 can be driven the stage driving mechanism 152 to move the stage 130 in the planar direction. The stage driving mechanism is composed of, for example, a motor, a shaft, a nut or the like. The fluorescent light generated from the fluorescent dye 51 on the substrate 80 passes through the objective lens 126 and penetrates through the dichroic mirror 125. The condenser lens 127 can condense fluorescent light that penetrates through the dichroic mirror 125 and guide the fluorescent light to a light-receiving surface 141 of the imaging unit 140. The imaging unit 140 can take an image of fluorescent light emitted to the light-receiving surface 141 to produce a fluorescent image. The imaging unit 140 is composed of, for example, a CCD or the like.

The information processing unit 102 is provided with a processing unit 161, a storage unit 162, a display unit 163, an input unit 164 and an interface 165.

The processing unit 161 is, for example, a CPU. The storage unit 162 is a ROM, a RAM, a hard disk or the like. The processing unit 161 can control each part of the information processing unit 102, the light source 111 in the light source unit 120, the imaging unit 140, the shutter driving mechanism 151 and the stage driving mechanism 152 through the interface 165 in accordance with a program stored in the storage unit 162.

The processing unit 161 can perform the information acquisition step shown in Fig. 11 in accordance with a program stored in the storage unit 162. Namely, in the information acquisition step, the processing unit 161 drives the light source 111 to receive fluorescent light generated from the fluorescent dye 51 by the imaging unit 140, and also drives the imaging unit 140 to acquire a fluorescent image. The processing unit 161 produces a super-resolved image on the basis of the fluorescent image acquired by the imaging unit 140. The processing unit 161 acquires information on the structure of the polypeptide 11 on the basis of the produced super-resolved image, and displays a screen including the acquired image on the display unit 163.

The display unit 163 is used for displaying processing results and the like produced by the processing unit 161, and the display unit 163 is a liquid crystal display, a plasma display, a cathode ray tube (CRT) display or the like. The display unit 163 displays a screen 90 shown in Fig. 13. The input unit 164 includes a keyboard and a mouse for receiving the input of a direction by an operator.

In the information acquisition step shown in Fig. 11, the polypeptide 11 on the substrate 80 is measured with a super-resolving fluorescence microscope having spatial resolution performance beyond the diffraction limit of light. Alternatively, the polypeptide 11 on the substrate 80 may also be measured with a Raman microscope, a probe microscope or an electron microscope. When both of a probe microscope and an electron microscope are used, it becomes possible to measure the polypeptide 11 with spatial resolution performance beyond the diffraction limit of light. In the case where a measurement utilizing fluorescent is not performed in the information acquisition step, e.g., the case where a Raman microscope or a probe microscope is used, the labeling of the polypeptide by the addition of the detection antibody is omitted. When the addition of the detection antibody is omitted, it becomes easier to bind the capture antibody to a biding site of the polypeptide 11 and therefore the polypeptide 11 can be immobilized on the substrate 80 more smoothly.

With respect to the information on the structure of the polypeptide, the size, morphology and aggregation degree of the polypeptide can be obtained when the polypeptide 11 is measured with a super-resolving fluorescence microscope, a Raman microscope, a probe microscope or an electron microscope. Particularly the structure of the polypeptide, among the information on the structure of the polypeptide, can be obtained by measuring the polypeptide 11 with a super-resolving fluorescence microscope, a Raman microscope or a probe microscope.

In the case where the polypeptide 11 is measured with a Raman microscope, a Raman spectrum reflecting molecules or atoms constituting the polypeptide 11 or an image reflecting the shape of the polypeptide 11 is acquired. Therefore, when a Raman microscope is used, the chemical bond can also be acquired in addition to the size, morphology, structure and aggregation degree of the polypeptide as the information on the structure of the polypeptide. More specifically, as the chemical bond of the polypeptide 11, the type, number, concentration, content ratio and the like of a molecule or atoms constituting the polypeptide 11 can be acquired. In this case, the chemical bond in the polypeptide 11 is also displayed, in addition to the size, morphology, structure or aggregation degree of the polypeptide 11, in a region 93 in the screen 90 shown in Fig. 13. For example, in the region 93, as the chemical bonds in the polypeptide 11, the followings are displayed: "the concentration of C=O is ... " and "the concentration of C-H is ...".

The present disclosure includes, within the scope thereof, a method for capturing an analyte. In the method, a polypeptide that is an analyte in a sample can be captured by carrying out the process from step S1 to step S4 shown in Fig. 1. Details about the steps are similar to those mentioned with respect to the method for acquiring information on an analyte according to the present embodiment. In the method for capturing an analyte according to the present embodiment, the polypeptide that is the analyte in the sample is captured by a capture substance to form a complex, and the complex is immobilized onto a second solid phase. The complex immobilized on the second solid phase is a measurement sample for acquiring information on the polypeptide that is the analyte. Accordingly, the method for capturing an analyte according to the present embodiment can also be referred to as a "method for preparing a measurement sample for acquiring information on an analyte". The method for capturing an analyte according to the present embodiment may also be referred to as a "method for pretreating a sample containing an analyte for the purpose of acquiring information on the analyte".

The present disclosure includes, within the scope thereof, a method for acquiring information on an analyte, including the step of acquiring information on a polypeptide that is an analyte from a complex containing the polypeptide and a capture substance capable of binding to the polypeptide. Details about the information acquisition step are similar to those mentioned with respect to the method for acquiring information on an analyte according to the present embodiment. In the method, a complex to be used for the measurement for information acquisition purpose can be obtained by the process from step S1 to step S4 shown in Fig. 1. Details about the individual steps are similar to those mentioned with respect to the method for acquiring information on an analyte according to the present embodiment.

Hereinafter, the present disclosure will be described in more detail with reference to examples.

### EXAMPLES

### Example 1

It was examined as to whether or not Aβ contained in a clinical specimen collected from a subject was able to be measured with high sensitivity by the method according to the present embodiment which utilized an immune complex transfer method (ICT) and a fluorescence microscope.

### 1. Preparation of sample

As a sample containing Aβ, a cerebrospinal fluid (CSF) collected from an Alzheimer's disease patient who agreed to the donation of a specimen was used. In Example 1, a pool CFS prepared by mixing CSFs collected from a plurality of patients(also referred to as a "clinical specimen 1", hereinafter) was used.

### 2. Preparation of reagents

### (2.1) Production of first solid phase

As a first solid phase, a magnetic particle having an anti-DNP antibody immobilized on the surface thereof (also simply referred to as a "magnetic particle", hereinafter) was produced in the following manner. An anti-DNP antibody (Sysmex Corporation) was immobilized onto a magnetic particle (product name: MAG2201, manufactured by JSR Corporation) having a particle diameter of 2.2 µm by a conventional technique. In this manner, a suspension of the magnetic particles each having the anti-DNP antibody immobilized on the surface thereof (particle concentration: 2.5%) was produced.

### (2.2) Production of second solid phase

As a second solid phase, a glass substrate having streptavidin immobilized on the surface thereof (also simply referred to as a "substrate", hereinafter) was produced in the following manner. Though-holes each having a diameter of 6 mm were formed in a silicon rubber sheet (SR-50, manufactured by Tigers Polymer Corporation), and then the silicon rubber sheet was attached to a MAS coat glass (Matsunami Glass Ind.,Ltd.). Thirty µg/mL-biotin-binding bovine serum albumin (BSA) (0.5 µL) was dropped onto a glass placed inside of the silicon rubber sheet, and then the glass was allowed to leave at room temperature for 1 hour. The glass was washed by pipetting with HISCL wash solution (Sysmex Corporation) (40 µL). The washing was carried out twice in total. The glass was further washed by pipetting using PBS (40 µL). The washing was carried out twice in total. A 1% B SA/PB S solution (40 µL) was dropped onto the glass, and the glass was allowed to leave at 4°C overnight. The glass was washed by pipetting with HISCL wash solution (Sysmex Corporation) (40 µL). The washing was carried out twice in total. The glass was further washed by pipetting using PBS (40 µL). The washing with PBS was carried out twice in total. A (10 µg/mL streptavidin)/(1% BSA/PBS) solution (40 µL) was dropped onto the glass, and the glass was stirred at room temperature for 1 hour. The glass was washed by pipetting with HISCL wash solution (Sysmex Corporation) (40 µL). The washing was carried out twice in total. The glass was further washed by pipetting using PBS (40 µL). The washing with PBS was carried out twice in total.

### (2.3) Capture substance

In Example 1, as a capture substance capable of binding to an Aβ peptide, a combination of a rabbit anti-amyloid β42 monoclonal antibody (Life Technologies Corporation, clone name: H31L21) (also referred to as "H31L21 antibody", hereinafter) and a murine anti-human amyloid β monoclonal antibody (IBL Co., Ltd. , clone name: 82E1) (also referred to as "82E1 antibody", hereinafter) was used. The H31L21 antibody is an antibody capable of binding to a C-terminal region of Aβ peptide and can recognize a region lying between position-36 and position-42 in Aβ. The 82E1 antibody is an antibody capable of binding to a N-terminal region in Aβ peptide, and can recognize a region lying between position-1 to position-16 in Aβ. In Example 1, DNP was used as a first bonding substance and biotin was used as a second bonding substance. More specifically, each of the antibodies was labeled with DNP or biotin, thereby producing a first capture substance and a second capture substance.

### (2.3.1) First capture substance having first bonding substance (DNP-labeled capture antibody)

Using N-succinimide S-acetylthioacetate (SATA) (Thermo Fischer Scientific), a thio group was introduced into each of the H31L21 antibody and the 82E1 antibody. Using N-(6-maleimidocaproyloxy)succinimide (EMCS), N-(2,4-dinitrophenyl)-L-lysine (DNP-Lys) (Tokyo Chemical Industry Co., Ltd.) was maleimdated. Each of the antibodies each having a thiol group introduced therein was mixed with and reacted with the maleimdated DNP-Lys. In this manner, as first capture substances for immobilizing Aβ onto the first solid phase (the above-mentioned magnetic particle), a DNP-labeled H31L21 antibody and a DNP-labeled 82E1 antibody were produced.

### (2.3.2) Second capture substance having second bonding substance (biotin-labeled capture antibody)

Using SATA (Thermo Fischer Scientific), a thiol group was introduced into each of the H31L21 antibody and the 82E1 antibody. Each of the antibodies each having a thiol group introduced therein was mixed with and reacted with Biotin-PEAC5-maleimide (6-[N'-[2-(N-maleimide)ethyl]-N-piperazinylamide]hexyl D-biotinamide hydrochloric acid salt). In this manner, as a second capture substance for immobilizing Aβ onto the second solid phase (the above-mentioned substrate), a biotin-labeled H31L21 antibody and a biotin-labeled 82E1 antibody were produced.

### (2.4) Substance for detection

A 82E1 antibody was labeled with a silylrhodamine-type fluorescent dye to produce a fluorescently labeled antibody for Aβ detection. The fluorescent dye was synthesized in accordance with the description of Grimm J.B. et. al., "A general method to improve fluorophores for live-cell and single-molecule microscopy", Nature Methods, vol.12, (2015) p. 244-250.

### (2.5) Releasing agent

As a releasing agent for dissociating the bonding between DNP in the first capture substance and the anti-DNP antibody in the first solid phase (magnetic particle), a 2.5-mM DNP-Lys solution was used. The DNP-Lys solution was prepared by diluting N-(2,4-dinitrophenyl)-L-lysine (Tokyo Chemical Industry Co., Ltd.) with a buffer (0.1 Tris-HCl (pH 7.5), 2% sodium caseinate, 0.1% NaN₃ and DMSO) so that the concentration became 2.5 mM.

### (2.6) Preparation of antibody solution

Any one of the two types of capture substances and any one of the two types of the substances for detection were mixed together in each of the combinations A to D shown in Table 1 in a HISCL R3 diluent (Sysmex Corporation) to produce an antibody solution. The antibody solutions were prepared in such a manner that the content of each of the antibodies in each of the antibody solutions became 300 fmol/assay. In the table, "(N)" represents the matter that the 82E1 antibody was an antibody capable of binding to a N-terminal region of Aβ, "(C)" represents the matter that the H31L21 antibody was an antibody capable of binding to a C-terminal region of Aβ. In Table 1, combination A of the antibodies corresponds to Fig. 2, and combination B of the antibodies corresponds to Fig. 7.

**[Table 1]**

| | Substance for detection | First capture substance | Second capture substance |
|---|---|---|---|
| | Fluorescently labeled antibody | DNP-labeled antibody | Biotin-labeled antibody |
| A | 82E1 (N) | H31L21 (C) | H31L21 (C) |
| B | 82E1(N) | H31L21 (C) | 82E1 (N) |
| C | 82E1(N) | 82E1 (N) | H31L21 (C) |
| D | 82E1(N) | 82E1 (N) | 82E1 (N) |

### 3. Measurement

Each of the antibody solutions, the first solid phase (magnetic particle) suspension, the HISCL wash solution and the releasing agent (DNP-Lys solution) were placed on Magtration System 6GC (Precision System Science Co., Ltd.) that was a full-automatic sample treatment device. The sample was treated by the device, and a complex containing Aβ and the antibodies was collected. Specific treatment was as follows. The sample (80 µL) was mixed with the antibody solution (80 µL), and the resultant mixed solution was incubated at 37°C for 30 minutes. The magnetic particle suspension (20 µL) was further mixed with the mixed solution, and the resultant solution was incubated at 37°C for 15 minutes. The magnetic particles were magnetically collected to remove a supernatant, and the HISCL wash solution (600 µL) was added thereto to wash the magnetic particles. The washing was carried out twice in total. The washing was carried out twice in total. Subsequently, the HISCL wash solution (150 µL) was further added to the washed magnetic particles to wash the magnetic particles. The washing was carried out once in total. After the washing, the DNP-Lys solution (30 µL) was added to the magnetic particles, and then the resultant solution was stirred at 37°C for 10 minutes. The magnetic particles were magnetically collected, and a supernatant was collected.

The collected supernatant was dropped onto the substrate, and was then stirred at room temperature for 3 hours. The substrate was washed by pipetting using a HISCL wash solution (Sysmex Corporation) (40 µL). The washing was carried out twice in total. The glass was further washed by pipetting using PBS (40 µL). The washing with PBS was carried out twice in total. A fluorescent image of Aβ on the substrate was taken with an inverted microscope equipped with a laser. Fluorescent images in 64 viewing fields were superposed on each other with Max intensity to obtain results.

### 4. Results

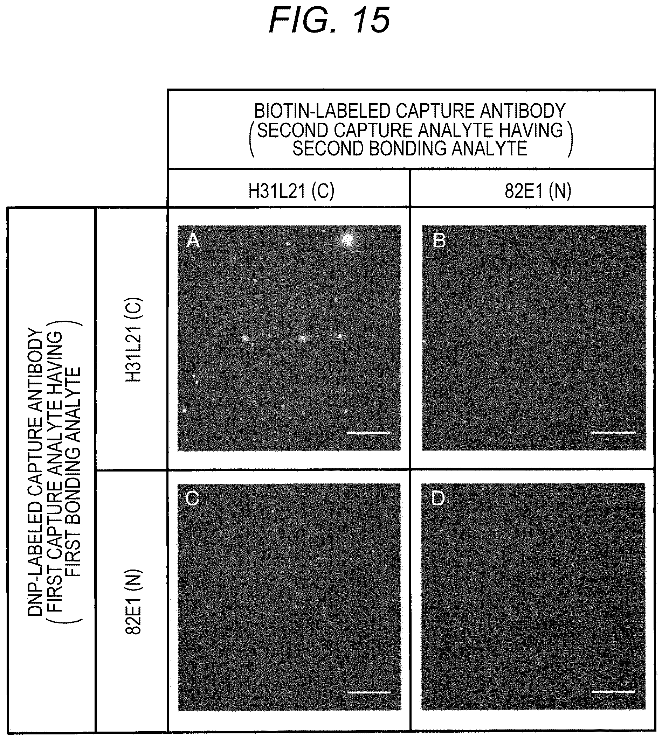

The obtained fluorescent images are shown in Fig. 15. In the drawings, "A", "B", "C" and "D" represent images obtained employing the antibody combinations A, B, C and D shown in Table 1, respectively. In the drawings, the scale bar indicates 10 µm. In Example 1, two or three of the fluorescently labeled antibody, the DNP-labeled antibody and the biotin-labeled antibody were the same clonal antibodies. Therefore, the bright points on the fluorescent images show Aβ aggregates of dimers or higher. The number of bright points in each of the fluorescent images in the clinical specimen 1 is shown in Table 2. The counting of the bright points was carried out by specifying, as a bright point region, a region having a pixel value larger than a given threshold value in a fluorescent image and then counting the number of the specified bright point regions.

**[Table 2]**

| | Substance for detection | First capture substance | Second capture substance | Number of bright points |
|---|---|---|---|---|
| | Fluorescently labeled antibody | DNP-labeled antibody | Biotin-labeled antibody | Clinical sample 1 |
| A | 82E1 (N) | H31L21 (C) | H31L21 (C) | 116 |
| B | 82E1 (N) | H31L21 (C) | 82E1 (N) | 18 |
| C | 82E1 (N) | 82E1 (N) | H31L21 (C) | 13 |
| D | 82E1 (N) | 82E1 (N) | 82E1 (N) | 2 |

As shown in Table 2, it was suggested that Aβ in a clinical specimen was able to be detected with high sensitivity when at least one of the first capture substance and the second capture substance was an antibody capable of binding to a C-terminal region of Aβ and the substance for detection was an antibody capable of binding to a N-terminal region of Aβ. Particularly when each of the first capture substance and the second capture substance was an antibody capable of binding to a C-terminal region of Aβ and the substance for detection was an antibody capable of binding to a N-terminal region of Aβ, the sensitivity of the detection of Aβ in a clinical specimen was significantly improved.

### Example 2

In Example 1, two type of capture substances were used in the ICT. In Example 2, it was examined as to whether or not Aβ contained in a clinical specimen was measured with high sensitivity by the method of the present embodiment utilizing an ICT using a single type of capture substance antibody and a fluorescence microscope.

### 1. Preparation of sample

As a sample containing Aβ, a cerebrospinal fluid (CSF) collected from an Alzheimer's disease patient who agreed to the donation of a specimen was used.

### 2. Preparation of reagents

In the measurement using two types of capture substances, as in the case of Example 1, a DNP-labeled H31L21 antibody was used as a first capture substance and a biotin-labeled H31L21 antibody was used as a second capture substance. In the measurement using a single type of capture substance, a H31L21 antibody labeled with both of DNP and biotin was used as the capture substance. A H31L21 antibody labeled with both of DNP and biotin was produced as mentioned below with reference to the description of WO 2017/138497 A1. A first solid phase, a second solid phase, a substance for detection (a fluorescently labeled 82E1 antibody) and a releasing agent were the same as those used in Example 1.

### (2.1) Production of capture substance having first and second bonding substances

A H31L21 antibody was digested with pepsin in the conventional manner to obtain a F(ab')₂ fragment. The F(ab')₂ fragment thus obtained was reduced to produce Fab'. BSA conjugated with DNP and biotin (product name: DNP-BSA-Biotin, LGC Biosearch Technologies) was reacted with a poly(ethylene glycol) (PEG) cross-linker (succinimidyl-[(N-maleimidepropionamide)-octa(ethylene glycol)] ester (product name: SM(PEG)₈, Thermo Fisher Scientific)) to bond a linker having a maleimide group to DNP-BSA-Biotin. The DNP-BSA-Biotin having the linker bonded thereto was mixed with the Fab' to cause the reaction of a thiol group in the Fab' with a maleimide group in the DNP-BSA-Biotin, thereby producing a DNP/biotin-labeled Fab'. In Example 2, a DNP/biotin-labeled Fab' derived from a H31L21 antibody was used as a DNP/biotin-labeled capture substance.

### (2.2) Preparation of antibody solution

Each of combinations of the capture substance and the substance for detection were mixed in a HISCL R3 diluent (Sysmex Corporation) in each of combinations of A and E shown in Table 3, thereby producing an antibody solution. The content of each of the antibodies in the antibody solution was adjusted to 300 fmol/assay. In Table 3, the antibody combination A corresponds to Fig. 2, and the antibody combination E corresponds to Fig. 5.

**[Table 3]**

| | Substance for detection | First capture substance | Second capture substance |
|---|---|---|---|
| | Fluorescently labeled antibody | DNP-labeled antibody | Biotin-labeled antibody |
| A | 82E1 (N) | H31L21 (C) | H31L21 (C) |

| | Substance for detection | Capture substance |
|---|---|---|
| | Fluorescently labeled antibody | DNP/biotin-labeled antibody |
| E | 82E1 (N) | H31L21 (C) |

### 3. Measurement

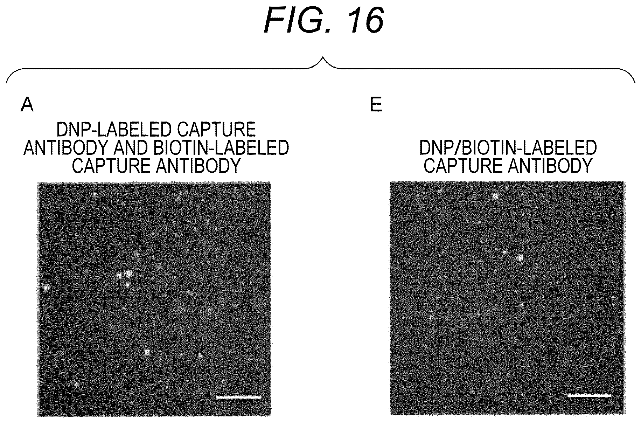

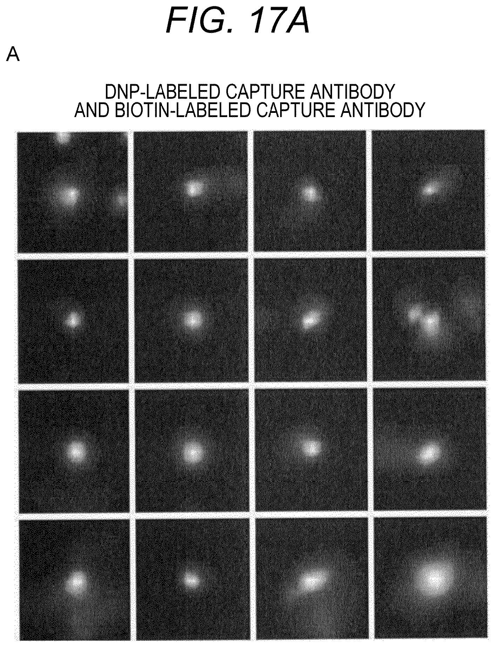

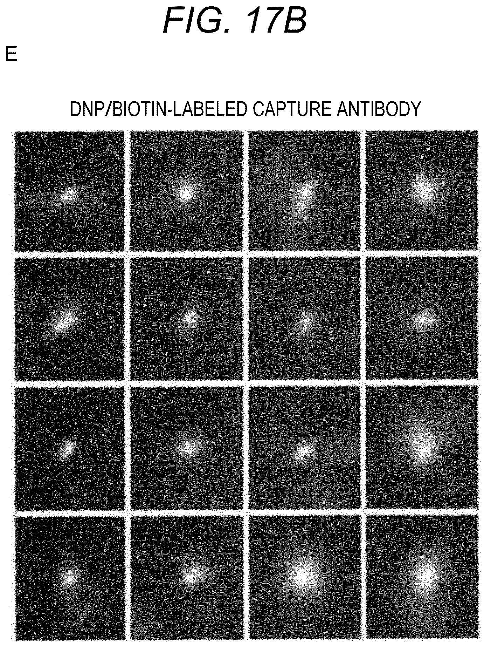

The measurement of each of the samples was carried out in the same manner as in Example 1, except that the above-mentioned antibody solutions were used. The measurement (n = 2) was carried out twice, and the measurement procedures were carried out independently. Typical examples of the acquired fluorescent images are shown in Fig. 16. Super-resolved images acquired with an inverted microscope are shown in Figs. 17A and 17B. In Figs. 16, 17A and 17B, "A" and "E" represent an image or images obtained using the antibody combination A and an image or images obtained using the antibody combination E in Table 3, respectively. In Fig. 16, the scale bar indicates 10 µm. In Figs. 17A and 17B, each panel had a size of 200 nm × 200 nm. The average values of the numbers of bright points in the fluorescent images are shown in Table 4.

**[Table 4]**

| | Substance for detection | First capture substance | Second capture substance | Number of bright points (average value) |
|---|---|---|---|---|
| | Fluorescently labeled antibody | DNP-labeled antibody | Biotin-labeled antibody | |
| A | 82E1 (N) | H31L21 (C) | H31L21 (C) | 42.5 |

| | Substance for detection | Capture substance | Number of bright points (average value) |
|---|---|---|---|
| | Fluorescently labeled antibody | DNP/biotin-labeled antibody | |
| E | 82E1 (N) | H31L21 (C) | 93.5 |

As shown in Table 4, it was demonstrated that, in the case where an antibody capable of binding to a C-terminal region of Aβ was used as a capture substance, Aβ in a clinical specimen was detected with high sensitivity regardless of the number of the capture substance, i.e., the use of a single type of capture substance or the use of two types of capture substances. As shown in each of panels in Figs. 17A and 17B, the size, morphology and aggregation degree of a mass of Aβ aggregates were identified with the resolution performance beyond the diffraction limit of light.

### Example 3

It was examined as to whether or not Aβ in a clinical specimen was able to be measured by the method according to the present embodiment utilizing chemiluminescent immunoassay (CELIA).

### 1. Preparation of sample

As a sample containing Aβ, a CSF collected from an Alzheimer's disease patient who agreed to the donation of a specimen (also referred to as a "clinical specimen 2", hereinafter) was used. As a positive control, a solution containing a reparation produced by chemically crosslinking aggregates of Beta Amyloid (1-42) (Anaspec) (wherein the preparation was referred to as "synthetic Aβ", hereinafter) at a concentration of 10 pg/mL was used. The chemical crosslinking was carried out in accordance with the description of Farid Rahimi et. al., "Photo-Induced Cross-Linking of Unmodified Proteins (PICUP) Applied to Amyloidogenic Peptides", J Vis Exp, vol.23, (2009), 1071. As a negative control, a C0 solution (Sysmex Corporation) was used.

### 2. Preparation of reagents

As a second solid phase, HISCL R2 reagent (Sysmex Corporation) which was a suspension containing magnetic particles each having streptavidin immobilized on the surface thereof was used. As a substance for detection, a 82E1 antibody labeled with alkaline phosphatase (ALP) was used. The ALP-labeled 82E1 antibody was produced as mentioned below in accordance with the description of WO 2017/138497 A1. A first solid phase, a first capture substance (a DNP-labeled H31L21 antibody), a second capture substance (a biotin-labeled H31L21 antibody and a biotin-labeled 82E1 antibody) and a releasing agent were the same as those used in Example 1. In the measurement of chemiluminescence, HISCL R4 reagent (Sysmex Corporation) which includes a measurement buffer and HISCL R5 reagent (Sysmex Corporation) which include CDP-Star (registered tradename) that was a chemiluminescent substrate for alkaline phosphatase were used.

### (2.1) Production of substance for detection (ALP-labeled detection antibody)

An 82E1 antibody was digested with pepsin by the conventional technique to obtain a F(ab')₂ fragment. The F(ab')₂ fragment thus obtained was reduced to produce Fab'. ALP was reacted with EMCS to maleimdate the ALP. The Fab' was mixed with the maleimdated ALP to cause the reaction of a thiol group in the Fab' with a maleimide group in the ALP, thereby producing an ALP-labeled Fab'. In Example 3, an ALP-labeled Fab' derived from a 82E1 antibody was used as the ALP-labeled detection antibody.

### (2.2) Preparation of antibody solution

Each of combinations of the capture substance and the substance for detection were mixed in a HISCL R3 diluent (Sysmex Corporation) in each of combinations of A and B shown in Table 5, thereby producing an antibody solution. The content of each of the antibodies in the antibody solution was adjusted to 300 fmol/assay. In Table 5, the antibody combination A corresponds to Fig. 2, and the antibody combination B corresponds to Fig. 7.

**[Table 5]**

| | Substance for detection | First capture substance | Second capture substance |
|---|---|---|---|
| | Fluorescently labeled antibody | DNP-labeled antibody | Biotin-labeled antibody |
| A | 82E1 (N) | H31L21 (C) | H31L21 (C) |
| B | 82E1 (N) | H31L21 (C) | 82E1 (N) |

### 3. Measurement

The measurement was carried out using a full-automatic immunoassay device HISCL-800 (Sysmex Corporation) in the following manner. A sample (70 µL) was mixed with an antibody solution (80 µL), and the resultant solution was incubated at 37°C for 27 minutes. The solution was further mixed with a suspension of a first solid phase (20 µL), and the resultant solution was incubated at 37°C for 11 minutes. The magnetic particles were magnetically collected, and then a supernatant was removed. A HISCL wash solution (300 µL) was added to the resultant product to wash the magnetic particles. The washing was carried out three times in total. After the washing, a DNP-Lys solution (110 µL) was added to the magnetic particles, and the resultant solution was incubated at 42°C for 5 minutes. The magnetic particles were magnetically collected, and a supernatant (80 µL) was collected. The collected supernatant was mixed with a second solid phase (HISCL R2 reagent) (30 µL), and the resultant solution was incubated at 37°C for 5 minutes. The magnetic particles in the mixed solution were magnetically collected, and a supernatant was removed. A HISCL wash solution (300 µL) was added to the resultant product to wash the magnetic particle. The washing was carried out four times in total. The magnetic particles were magnetically collected, and a supernatant was removed. The magnetic particles were mixed with HISCL R4 reagent (30 µL) and HISCL R5 reagent (60 µL), then the resultant solution was incubated at 42°C for 5 minutes, and then the luminous intensity of the solution was measured.

### 4. Results

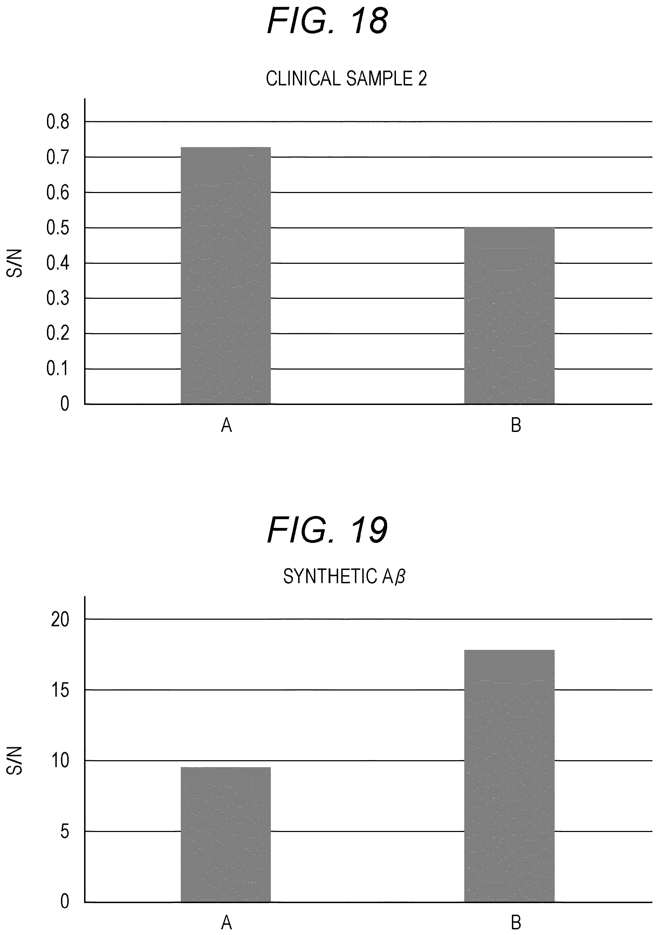

All of the measurement values of the chemiluminescence intensities exceeded the detection limit. An SN ratio (S/N) was calculated from each of the measurement values in accordance with the below-mentioned formula. The results are shown in Figs. 18 and 19. In Figs. 18 and 19, "A" and "B" represent an image or images obtained using the antibody combination A shown in Table 5 and an image or images obtained using the antibody combination B shown in Table 5, respectively. (S/N) = [(luminous intensity of sample)-(luminous intensity of negative control)]/(luminous intensity of negative control)

As shown in Figs. 18 and 19, Aβ in a sample was measured by the method of the present embodiment utilizing CELIA. As apparent from Fig. 18, an S/N ratio obtained employing the antibody combination A shown in Table 5 was higher than that obtained employing the antibody combination B shown in Table 5. **In** contrast, as shown in Fig. 19, when synthetic Aβ was measure, a S/N ratio obtained employing the antibody combination B shown in Table 5 was higher than that obtained employing the antibody combination A shown in Table 5. From these results, it was suggested that, when a sample is a clinical specimen, Aβ can be detected with higher sensitivity by using antibodies each capable of binding to a C-terminal region of Aβ as the first capture substance and the second capture substance and using an antibody capable of binding to a N-terminal region of Aβ as the substance for detection.

## Claims

1. A method for acquiring information on an analyte, comprising the steps of:
bringing a polypeptide that is the analyte into contact with a capture substance that binds to the polypeptide to form a complex comprising the polypeptide and the capture substance;
bonding the capture substance in the complex to a first solid phase to immobilize the complex onto the first solid phase;
collecting the first solid phase having the complex immobilized on the first solid phase;
releasing the complex from the collected first solid phase, and then bonding the capture substance in the released complex to a second solid phase to immobilize the complex onto the second solid phase; and
acquiring information on the polypeptide from the complex immobilized on the second solid phase,
wherein the capture substance that binds to at least one of the first solid phase and the second solid phase binds to a C-terminal region of the polypeptide and is a H31L21 antibody,
wherein the capture substance comprises a first capture substance having a first bonding substance and a second capture substance having a second bonding substance, and at least one of the first capture substance and the second capture substance binds to a C-terminal region of the polypeptide;
the first solid phase has a first bonding partner that bonds specifically to the first bonding substance and the second solid phase has a second bonding partner that bonds specifically to the second bonding substance;
in the step of immobilizing the complex onto the first solid phase, the complex is immobilized onto the first solid phase through a specific bonding between the first bonding substance and the first bonding partner; and
in the step of immobilizing the complex onto the second solid phase, the complex is immobilized onto the second solid phase through a specific bonding between the second bonding substance and the second bonding partner,
wherein the polypeptide is further brought into contact with a substance for detection which has a labeling substance and binds to the polypeptide to form a complex comprising the polypeptide, the capture substance and the substance for detection, wherein the substance for detection binds to a N-terminal region of the polypeptide, and wherein the polypeptide is amyloid β.

2. The method according to claim 1, wherein the capture substance that binds to the first solid phase binds to a C-terminal region of the polypeptide.

3. The method according to claim 1 or 2, wherein the first solid phase is a magnetic particle.

4. The method according to any one of claims 1 to 3, wherein the capture substance that binds to the second solid phase binds to a C-terminal region of the polypeptide.

5. The method according to any one of claims 1 to 4, wherein the second solid phase is a substrate.

6. The method according to claim 5, wherein the substance for detection comprises an 82E1 antibody.

7. The method according to claim 1, wherein the first capture substance has a first bonding substance and binds to a C-terminal region of the polypeptide.

8. The method according to any one of claims 1 to 6, wherein the capture substance has both of a first bonding substance and a second bonding substance and binds to a C-terminal region of the polypeptide;
the first solid phase has a first bonding partner that bonds specifically to the first bonding substance, and the second solid phase has a second bonding partner that bonds specifically to the second bonding substance;
in the step of immobilizing the complex onto the first solid phase, the complex is immobilized onto the first solid phase through a specific bonding between the first bonding substance and the first bonding partner; and
in the step of immobilizing the complex onto the second solid phase, the complex is immobilized onto the second solid phase through a specific bonding between the second bonding substance and the second bonding partner.

9. The method according to any one of claims 1 to 8, wherein the first bonding substance is a hapten and the first bonding partner is an anti-hapten antibody.

10. The method according to any one of claims 1 to 9, wherein the second bonding substance is a biotin-type compound and the second bonding partner is an avidin-type compound.

11. The method according to any one of claims 1 to 10, wherein the information on the polypeptide is information on a structure of the polypeptide.

12. The method according to any one of claims 1 to 10, wherein the information on the polypeptide is information on at least one of a size, morphology and an aggregation degree of the polypeptide.

13. The method according to claim 11 or 12, wherein, in the information acquisition step, the polypeptide on the first solid phase is imaged with a microscope to acquire an image of the polypeptide.

14. The method according to claim 13, wherein the microscope is a fluorescence microscope, a super-resolving microscope, a Raman microscope, a probe microscope or an electron microscope.

15. The method according to any one of claims 1 to 10, wherein the information on the polypeptide is information on a quantity of the polypeptide.

16. The method according to any one of claim 1 and claims 7 to 15 which refer to claim 1 directly or indirectly, wherein the labeling substance in the substance for detection is an enzyme, a fluorescent substance or a radioactive isotope.

17. The method according to claim 16, wherein the labeling substance in the substance for detection is an enzyme; and
in the information acquisition step, the enzyme in the complex is reacted with a substrate for the enzyme and a signal generated from a reaction product produced by the enzymatic reaction is measured.

18. The method according to claim 16, wherein the labeling substance in the substance for detection is a fluorescent substance; and
in the information acquisition step, the complex is irradiated with excitation light and a fluorescence generated from the fluorescent substance in the complex is measured.

19. A method for capturing an analyte, comprising the steps of:
bringing a polypeptide that is the analyte into contact with a capture substance that binds to the polypeptide to form a complex comprising the polypeptide and the capture substance;
bonding the capture substance in the complex to a first solid phase to immobilize the complex onto the first solid phase;
collecting the first solid phase having the complex immobilized on the first solid phase; and
releasing the complex from the collected first solid phase, and then bonding the capture substance in the released complex to a second solid phase to immobilize the complex onto the second solid phase,
wherein the capture substance that binds to at least one of the first solid phase and the second solid phase binds to a C-terminal region of the polypeptide, and is a H31L21 antibody,
wherein the capture substance comprises a first capture substance having a first bonding substance and a second capture substance having a second bonding substance, and at least one of the first capture substance and the second capture substance binds to a C-terminal region of the polypeptide;
the first solid phase has a first bonding partner that bonds specifically to the first bonding substance and the second solid phase has a second bonding partner that bonds specifically to the second bonding substance;
in the step of immobilizing the complex onto the first solid phase, the complex is immobilized onto the first solid phase through a specific bonding between the first bonding substance and the first bonding partner; and
in the step of immobilizing the complex onto the second solid phase, the complex is immobilized onto the second solid phase through a specific bonding between the second bonding substance and the second bonding partner,
wherein the polypeptide is further brought into contact with a substance for detection which has a labeling substance and binds to the polypeptide to form a complex comprising the polypeptide, the capture substance and the substance for detection, wherein the substance for detection binds to a N-terminal region of the polypeptide, and wherein the polypeptide is amyloid β.

20. A method for acquiring information on an analyte, comprising the step of acquiring information of a polypeptide that is the analyte from a complex comprising the polypeptide and a capture substance that binds to the polypeptide,
wherein
the complex is produced by:
bringing the polypeptide into contact with the capture substance to form a complex comprising the polypeptide and the capture substance;
bonding the capture substance in the complex to a first solid phase to immobilize the complex onto the first solid phase; collecting the first solid phase having the complex immobilized on the first solid phase; and releasing the complex from the collected first solid phase, and then bonding the capture substance in the released complex to a second solid phase,
wherein the capture substance that binds to at least one of the first solid phase and the second solid phase binds to a C-terminal region of the polypeptide, and is a H31L21 antibody,
wherein the capture substance comprises a first capture substance having a first bonding substance and a second capture substance having a second bonding substance, and at least one of the first capture substance and the second capture substance binds to a C-terminal region of the polypeptide;
the first solid phase has a first bonding partner that bonds specifically to the first bonding substance and the second solid phase has a second bonding partner that bonds specifically to the second bonding substance;
in the step of immobilizing the complex onto the first solid phase, the complex is immobilized onto the first solid phase through a specific bonding between the first bonding substance and the first bonding partner; and
in the step of immobilizing the complex onto the second solid phase, the complex is immobilized onto the second solid phase through a specific bonding between the second bonding substance and the second bonding partner,
wherein the polypeptide is further brought into contact with a substance for detection which has a labeling substance and binds to the polypeptide to form a complex comprising the polypeptide, the capture substance and the substance for detection, wherein the substance for detection binds to a N-terminal region of the polypeptide, and wherein the polypeptide is amyloid β.

## Patentansprüche

1. Verfahren zum Erlangen von Informationen über einen Analyten, umfassend die Schritte:
Inkontaktbringen eines Polypeptids, welches der Analyt ist, mit einer Einfangsubstanz, die an das Polypeptid bindet unter Bildung eines Komplexes, welcher das Polypeptid und die Einfangsubstanz umfasst;
Binden der Einfangsubstanz in dem Komplex an eine erste feste Phase, um den Komplex auf der ersten festen Phase zu immobilisieren;
Gewinnen der ersten festen Phase, die den auf der ersten festen Phase immobilisierten Komplex aufweist;
Freisetzen des Komplexes aus der gewonnenen ersten festen Phase und dann Binden der Einfangsubstanz in dem freigesetzten Komplex an eine zweite feste Phase, um den Komplex auf der zweiten festen Phase zu immobilisieren; und
Erlangen von Informationen über das Polypeptid aus dem auf der zweiten festen Phase immobilisierten Komplex,
wobei die Einfangsubstanz, die an wenigstens eine von der ersten festen Phase und der zweiten festen Phase bindet, an eine C-terminale Region des Polypeptids bindet und ein H31L21-Antikörper ist,
wobei die Einfangsubstanz eine erste Einfangsubstanz mit einer ersten Bindesubstanz und eine zweite Einfangsubstanz mit einer zweiten Bindesubstanz umfasst und wenigstens eine von der ersten Einfangsubstanz und der zweiten Einfangsubstanz an eine C-terminale Region des Polypeptids bindet;
die erste feste Phase einen ersten Bindungspartner hat, der spezifisch an die erste Bindesubstanz bindet, und die zweite feste Phase einen zweiten Bindungspartner hat, der spezifisch an die zweite Bindesubstanz bindet;
in dem Schritt des Immobilisierens des Komplexes auf der ersten festen Phase der Komplex durch eine spezifische Bindung zwischen der ersten Bindesubstanz und dem ersten Bindungspartner auf der ersten festen Phase immobilisiert wird; und
in dem Schritt des Immobilisierens des Komplexes auf der zweiten festen Phase der Komplex durch eine spezifische Bindung zwischen der zweiten Bindesubstanz und dem zweiten Bindungspartner auf der zweiten festen Phase immobilisiert wird, wobei das Polypeptid ferner mit einer Substanz zur Detektion in Kontakt gebracht wird, die eine Markierungssubstanz aufweist und das Polypeptid bindet unter Bildung eines Komplexes, welcher das Polypeptid, die Einfangsubstanz und die Substanz zur Detektion umfasst, wobei die Substanz zur Detektion an eine N-terminale Region des Polypeptids bindet und wobei das Polypeptid Amyloid β ist.

2. Verfahren gemäß Anspruch 1, wobei die Einfangsubstanz, die an die erste feste Phase bindet, an eine C-terminale Region des Polypeptids bindet.

3. Verfahren gemäß Anspruch 1 oder 2, wobei die erste feste Phase ein magnetisches Partikel ist.

4. Verfahren gemäß irgendeinem der Ansprüche 1 bis 3, wobei die Einfangsubstanz, die an die zweite feste Phase bindet, an eine C-terminale Region des Polypeptids bindet.

5. Verfahren gemäß irgendeinem der Ansprüche 1 bis 4, wobei die zweite feste Phase ein Substrat ist.

6. Verfahren gemäß Anspruch 5, wobei die Substanz zur Detektion einen 82E1-Antikörper umfasst.

7. Verfahren gemäß Anspruch 1, wobei die erste Einfangsubstanz eine erste Bindesubstanz aufweist und an eine C-terminale Region des Polypeptids bindet.

8. Verfahren gemäß irgendeinem der Ansprüche 1 bis 6, wobei die Einfangsubstanz sowohl eine erste Bindesubstanz als auch eine zweite Bindesubstanz aufweist und an eine C-terminale Region des Polypeptids bindet;
die erste feste Phase einen ersten Bindungspartner hat, der spezifisch an die erste Bindesubstanz bindet, und die zweite feste Phase einen zweiten Bindungspartner hat, der spezifisch an die zweite Bindesubstanz bindet;
in dem Schritt des Immobilisierens des Komplexes auf der ersten festen Phase der Komplex durch eine spezifische Bindung zwischen der ersten Bindesubstanz und dem ersten Bindungspartner auf der ersten festen Phase immobilisiert wird; und
in dem Schritt des Immobilisierens des Komplexes auf der zweiten festen Phase der Komplex durch eine spezifische Bindung zwischen der zweiten Bindesubstanz und dem zweiten Bindungspartner auf der zweiten festen Phase immobilisiert wird.

9. Verfahren gemäß irgendeinem der Ansprüche 1 bis 8, wobei die erste Bindesubstanz ein Hapten ist und der erste Bindungspartner ein Anti-Hapten-Antikörper ist.

10. Verfahren gemäß irgendeinem der Ansprüche 1 bis 9, wobei die zweite Bindesubstanz eine Verbindung des Biotintyps ist und der zweite Bindungspartner eine Verbindung des Avidintyps ist.

11. Verfahren gemäß irgendeinem der Ansprüche 1 bis 10, wobei die Informationen über das Polypeptid Informationen über eine Struktur des Polypeptids sind.

12. Verfahren gemäß irgendeinem der Ansprüche 1 bis 10, wobei die Informationen über das Polypeptid Informationen über wenigstens eine/n von einer Größe, einer Morphologie und einem Aggregationsgrad des Polypeptids sind.

13. Verfahren gemäß Anspruch 11 oder 12, wobei in dem Schritt des Erlangens von Informationen das Polypeptid auf der ersten festen Phase mit einem Mikroskop abgebildet wird, um eine Abbildung des Polypeptids zu erlangen.

14. Verfahren gemäß Anspruch 13, wobei das Mikroskop ein Fluoreszenzmikroskop, ein hochauflösendes Mikroskop, ein Raman-Mikroskop, ein Sondenmikroskop oder ein Elektronenmikroskop ist.

15. Verfahren gemäß irgendeinem der Ansprüche 1 bis 10, wobei die Informationen über das Polypeptid Informationen über eine Menge des Polypeptids sind.

16. Verfahren gemäß irgendeinem von Anspruch 1 und den Ansprüchen 7 bis 15, die sich direkt oder indirekt auf Anspruch 1 beziehen, wobei die Markierungssubstanz in der Substanz zur Detektion ein Enzym, eine fluoreszierende Substanz oder ein radioaktives Isotop ist.

17. Verfahren gemäß Anspruch 16, wobei die Markierungssubstanz in der Substanz zur Detektion ein Enzym ist; und
in dem Schritt des Erlangens von Informationen das Enzym in dem Komplex mit einem Substrat für das Enzym reagieren gelassen wird und ein aus einem durch die enzymatische Reaktion produzierten Reaktionsprodukt erzeugtes Signal gemessen wird.

18. Verfahren gemäß Anspruch 16, wobei die Markierungssubstanz in der Substanz zur Detektion eine fluoreszierende Substanz ist; und
in dem Schritt des Erlangens von Informationen der Komplex mit Anregungslicht bestrahlt wird und eine aus der fluoreszierenden Substanz in dem Komplex erzeugte Fluoreszenz gemessen wird.

19. Verfahren zum Einfangen eines Analyten, umfassend die Schritte:
Inkontaktbringen eines Polypeptids, welches der Analyt ist, mit einer Einfangsubstanz, die an das Polypeptid bindet unter Bildung eines Komplexes, welcher das Polypeptid und die Einfangsubstanz umfasst;
Binden der Einfangsubstanz in dem Komplex an eine erste feste Phase, um den Komplex auf der ersten festen Phase zu immobilisieren;
Gewinnen der ersten festen Phase, die den auf der ersten festen Phase immobilisierten Komplex aufweist;
Freisetzen des Komplexes aus der gewonnenen ersten festen Phase und dann Binden der Einfangsubstanz in dem freigesetzten Komplex an eine zweite feste Phase, um den Komplex auf der zweiten festen Phase zu immobilisieren,
wobei die Einfangsubstanz, die an wenigstens eine von der ersten festen Phase und der zweiten festen Phase bindet, an eine C-terminale Region des Polypeptids bindet und ein H31L21-Antikörper ist,
wobei die Einfangsubstanz eine erste Einfangsubstanz mit einer ersten Bindesubstanz und eine zweite Einfangsubstanz mit einer zweiten Bindesubstanz umfasst und wenigstens eine von der ersten Einfangsubstanz und
der zweiten Einfangsubstanz an eine C-terminale Region des Polypeptids bindet;
die erste feste Phase einen ersten Bindungspartner hat, der spezifisch an die erste Bindesubstanz bindet, und die zweite feste Phase einen zweiten Bindungspartner hat, der spezifisch an die zweite Bindesubstanz bindet;
in dem Schritt des Immobilisierens des Komplexes auf der ersten festen Phase der Komplex durch eine spezifische Bindung zwischen der ersten Bindesubstanz und dem ersten Bindungspartner auf der ersten festen Phase immobilisiert wird; und
in dem Schritt des Immobilisierens des Komplexes auf der zweiten festen Phase der Komplex durch eine spezifische Bindung zwischen der zweiten Bindesubstanz und dem zweiten Bindungspartner auf der zweiten festen Phase immobilisiert wird, wobei das Polypeptid ferner mit einer Substanz zur Detektion in Kontakt gebracht wird, die eine Markierungssubstanz aufweist und das Polypeptid bindet unter Bildung eines Komplexes, welcher das Polypeptid, die Einfangsubstanz und die Substanz zur Detektion umfasst, wobei die Substanz zur Detektion an eine N-terminale Region des Polypeptids bindet und wobei das Polypeptid Amyloid β ist.

20. Verfahren zum Erlangen von Informationen über einen Analyten, umfassend den Schritt des Erlangens von Informationen eines Polypeptids, welches der Analyt ist, aus einem Komplex, welcher das Polypeptid und eine Einfangsubstanz, die an das Polypeptid bindet, umfasst, wobei
der Komplex produziert wird durch:
Inkontaktbringen des Polypeptids mit der Einfangsubstanz unter Bildung eines Komplexes, welcher das Polypeptid und die Einfangsubstanz umfasst;
Binden der Einfangsubstanz in dem Komplex an eine erste feste Phase, um den Komplex auf der ersten festen Phase zu immobilisieren; Gewinnen der ersten festen Phase, die den auf der ersten festen Phase immobilisierten Komplex aufweist; und Freisetzen des Komplexes aus der gewonnenen ersten festen Phase und dann Binden der Einfangsubstanz in dem freigesetzten Komplex an eine zweite feste Phase,
wobei die Einfangsubstanz, die an wenigstens eine von der ersten festen Phase und der zweiten festen Phase bindet, an eine C-terminale Region des Polypeptids bindet und ein H31L21-Antikörper ist,
wobei die Einfangsubstanz eine erste Einfangsubstanz mit einer ersten Bindesubstanz und eine zweite Einfangsubstanz mit einer zweiten Bindesubstanz umfasst und wenigstens eine von der ersten Einfangsubstanz und der zweiten Einfangsubstanz an eine C-terminale Region des Polypeptids bindet;
die erste feste Phase einen ersten Bindungspartner hat, der spezifisch an die erste Bindesubstanz bindet, und die zweite feste Phase einen zweiten Bindungspartner hat, der spezifisch an die zweite Bindesubstanz bindet;
in dem Schritt des Immobilisierens des Komplexes auf der ersten festen Phase der Komplex durch eine spezifische Bindung zwischen der ersten Bindesubstanz und dem ersten Bindungspartner auf der ersten festen Phase immobilisiert wird; und
in dem Schritt des Immobilisierens des Komplexes auf der zweiten festen Phase der Komplex durch eine spezifische Bindung zwischen der zweiten Bindesubstanz und dem zweiten Bindungspartner auf der zweiten festen Phase immobilisiert wird, wobei das Polypeptid ferner mit einer Substanz zur Detektion in Kontakt gebracht wird, die eine Markierungssubstanz aufweist und das Polypeptid bindet unter Bildung eines Komplexes, welcher das Polypeptid, die Einfangsubstanz und die Substanz zur Detektion umfasst, wobei die Substanz zur Detektion an eine N-terminale Region des Polypeptids bindet und wobei das Polypeptid Amyloid β ist.

## Revendications

1. Procédé d'acquisition d'informations sur un analyte, comprenant les étapes consistant à :
amener un polypeptide qui est l'analyte en contact avec une substance de capture qui se lie au polypeptide afin de former un complexe comprenant le polypeptide et la substance de capture ;
lier la substance de capture dans le complexe à une première phase solide afin d'immobiliser le complexe sur la première phase solide ;
collecter la première phase solide présentant le complexe immobilisé sur la première phase solide ;
libérer le complexe de la première phase solide collectée, puis lier la substance de capture dans le complexe libéré à une seconde phase solide pour immobiliser le complexe sur la seconde phase solide ; et
acquérir des informations concernant le polypeptide à partir du complexe immobilisé sur la seconde phase solide,
dans lequel la substance de capture qui se lie à au moins une parmi la première phase solide et la seconde phase solide se lie à une région de terminaison C du polypeptide et est un anticorps H31L21,
dans lequel la substance de capture comprend une première substance de capture présentant une première substance de liaison et une seconde substance de capture présentant une seconde substance de liaison, et au moins une parmi la première substance de capture et la seconde substance de capture se lie à une région de terminaison C du polypeptide ;
la première phase solide présente un premier partenaire de liaison qui se lie spécifiquement à la première substance de liaison et la seconde phase solide présente un second partenaire de liaison qui se lie spécifiquement à la seconde substance de liaison ;
à l'étape d'immobilisation du complexe sur la première phase solide, le complexe est immobilisé sur la première phase solide par le biais d'une liaison spécifique entre la première substance de liaison et le premier partenaire de liaison ; et
à l'étape d'immobilisation du complexe sur la seconde phase solide, le complexe est immobilisé sur la seconde phase solide par le biais d'une liaison spécifique entre la seconde substance de liaison et le second partenaire de liaison, dans lequel le polypeptide est en outre amené en contact avec une substance pour détection qui présente une substance d'étiquetage et se lie au polypeptide pour former un complexe comprenant le polypeptide, la substance de capture et la substance pour détection, dans lequel la substance pour détection se lie à une région de terminaison N du polypeptide, et dans lequel le polypeptide est le β-amyloïde.

2. Procédé selon la revendication 1, dans lequel la substance de capture qui se lie à la première phase solide se lie à une région de terminaison C du polypeptide.

3. Procédé selon la revendication 1 ou 2, dans lequel la première phase solide est une particule magnétique.

4. Procédé selon l'une quelconque des revendications 1 à 3, dans lequel la substance de capture qui se lie à la seconde phase solide se lie à une région de terminaison C du polypeptide.

5. Procédé selon l'une quelconque des revendications 1 à 4, dans lequel la seconde phase solide est un substrat.

6. Procédé selon la revendication 5, dans lequel la substance pour détection comprend un anticorps 82E1.

7. Procédé selon la revendication 1, dans lequel la première substance de capture présente une première substance de liaison et se lie à une région de terminaison C du polypeptide.

8. Procédé selon l'une quelconque des revendications 1 à 6, dans lequel la substance de capture présente à la fois une première substance de liaison et une seconde substance de liaison et se lie à une région de terminaison C du polypeptide ;
la première phase solide présente un premier partenaire de liaison qui se lie spécifiquement à la première substance de liaison, et la seconde phase solide présente un second partenaire de liaison qui se lie spécifiquement à la seconde substance de liaison ;
à l'étape d'immobilisation du complexe sur la première phase solide, le complexe est immobilisé sur la première phase solide par le biais d'une liaison spécifique entre la première substance de liaison et le premier partenaire de liaison ; et
à l'étape d'immobilisation du complexe sur la seconde phase solide, le complexe est immobilisé sur la seconde phase solide par le biais d'une liaison spécifique entre la seconde substance de liaison et le second partenaire de liaison.

9. Procédé selon l'une quelconque des revendications 1 à 8, dans lequel la première substance de liaison est un haptène et le premier partenaire de liaison est un anticorps anti-haptène.

10. Procédé selon l'une quelconque des revendications 1 à 9, dans lequel la seconde substance de liaison est un composé de type biotine et le second partenaire de liaison est un composé de type avidine.

11. Procédé selon l'une quelconque des revendications 1 à 10, dans lequel les informations concernant le polypeptide sont des informations concernant une structure du polypeptide.

12. Procédé selon l'une quelconque des revendications 1 à 10, dans lequel les informations concernant le polypeptide sont des informations concernant au moins l'un parmi une taille, une morphologie et un degré d'agrégation du polypeptide.

13. Procédé selon la revendication 11 ou 12, dans lequel, à l'étape d'acquisition d'informations, le polypeptide concernant la première phase solide est imagé à l'aide d'un microscope afin d'acquérir une image du polypeptide.

14. Procédé selon la revendication 13, dans lequel le microscope est un microscope à fluorescence, un microscope à super-résolution, un microscope Raman, un microscope à sonde ou un microscope électronique.

15. Procédé selon l'une quelconque des revendications 1 à 10, dans lequel les informations concernant le polypeptide sont des informations concernant une quantité du polypeptide.

16. Procédé selon l'une quelconque de la revendication 1 et des revendications 7 à 15 qui se rapportent à la revendication 1 directement ou indirectement, dans lequel la substance d'étiquetage dans la substance pour détection est une enzyme, une substance fluorescente ou un isotope radioactif.

17. Procédé selon la revendication 15, dans lequel la substance d'étiquetage dans la substance pour détection est une enzyme ; et
à l'étape d'acquisition d'informations, l'enzyme dans le complexe est mise en réaction avec un substrat pour l'enzyme et un signal généré à partir d'un produit de réaction produit par la réaction enzymatique est mesuré.

18. Procédé selon la revendication 15, dans lequel la substance d'étiquetage dans la substance pour détection est une substance fluorescente ; et
à l'étape d'acquisition d'informations, le complexe est irradié avec une lumière d'excitation et une fluorescence générée à partir de la substance fluorescente dans le complexe est mesurée.

19. Procédé de capture d'un analyte, comprenant les étapes consistant à :
amener un polypeptide qui est l'analyte en contact avec une substance de capture qui se lie au polypeptide afin de former un complexe comprenant le polypeptide et la substance de capture ;
lier la substance de capture dans le complexe à une première phase solide afin d'immobiliser le complexe sur la première phase solide ;
collecter la première phase solide présentant le complexe immobilisé sur la première phase solide ; et
libérer le complexe de la première phase solide collectée, et ensuite lier la substance de capture dans le complexe libéré à une seconde phase solide pour immobiliser le complexe sur la seconde phase solide,
dans lequel la substance de capture qui se lie à au moins une parmi la première phase solide et la seconde phase solide se lie à une région de terminaison C du polypeptide, et est un anticorps H31L21,
dans lequel la substance de capture comprend une première substance de capture présentant une première substance de liaison et une seconde substance de capture présentant une seconde substance de liaison, et au moins une parmi la première substance de capture et la seconde substance de capture se lie à une région de terminaison C du polypeptide ;
la première phase solide présente un premier partenaire de liaison qui se lie spécifiquement à la première substance de liaison et la seconde phase solide présente un second partenaire de liaison qui se lie spécifiquement à la seconde substance de liaison ;
à l'étape d'immobilisation du complexe sur la première phase solide, le complexe est immobilisé sur la première phase solide par le biais d'une liaison spécifique entre la première substance de liaison et le premier partenaire de liaison ; et
à l'étape d'immobilisation du complexe sur la seconde phase solide, le complexe est immobilisé sur la seconde phase solide par le biais d'une liaison spécifique entre la seconde substance de liaison et le second partenaire de liaison, dans lequel
le polypeptide est en outre amené en contact avec une substance pour détection qui présente une substance d'étiquetage et se lie au polypeptide pour former un complexe comprenant le polypeptide, la substance de capture et la substance pour détection, dans lequel la substance pour détection se lie à une région de terminaison N du polypeptide, et dans lequel le polypeptide est le β-amyloïde.

20. Procédé d'acquisition d'informations sur un analyte, comprenant l'étape d'acquisition d'informations d'un polypeptide qui est l'analyte à partir d'un complexe comprenant le polypeptide et une substance de capture qui se lie au polypeptide,
dans lequel
le complexe est produit en :
amenant le polypeptide en contact avec la substance de capture afin de former un complexe comprenant le polypeptide et la substance de capture ;
liant la substance de capture dans le complexe à une première phase solide afin d'immobiliser le complexe sur la première phase solide ; collectant la première phase solide présentant le complexe immobilisé sur la première phase solide ; et libérant le complexe de la première phase solide collectée, puis liant la substance de capture dans le complexe libéré à une seconde phase solide,
dans lequel la substance de capture qui se lie à au moins une parmi la première phase solide et la seconde phase solide se lie à une région de terminaison C du polypeptide et est un anticorps H31L21, dans lequel
la substance de capture comprend une première substance de capture présentant une première substance de liaison et une seconde substance de capture présentant une seconde substance de liaison, et au moins une parmi la première substance de capture et la seconde substance de capture se lie à une région de terminaison C du polypeptide ;
la première phase solide présente un premier partenaire de liaison qui se lie spécifiquement à la première substance de liaison et la seconde phase solide présente un second partenaire de liaison qui se lie spécifiquement à la seconde substance de liaison ;
à l'étape d'immobilisation du complexe sur la première phase solide, le complexe est immobilisé sur la première phase solide par le biais d'une liaison spécifique entre la première substance de liaison et le premier partenaire de liaison ; et
à l'étape d'immobilisation du complexe sur la seconde phase solide, le complexe est immobilisé sur la seconde phase solide par le biais d'une liaison spécifique entre la seconde substance de liaison et le second partenaire de liaison, dans lequel
le polypeptide est en outre amené en contact avec une substance pour détection qui présente une substance d'étiquetage et se lie au polypeptide pour former un complexe comprenant le polypeptide, la substance de capture et la substance pour détection, dans lequel la substance pour détection se lie à une région de terminaison N du polypeptide, et dans lequel le polypeptide est le β-amyloïde.
